(19) Europäisches Patentamt
European Patent Office
Office européen des brevets

(11) EP 3 820 845 B1

(12) **EUROPEAN PATENT SPECIFICATION**

(45) Date of publication and mention of the grant of the patent:
**24.05.2023 Bulletin 2023/21**

(21) Application number: **19901368.1**

(22) Date of filing: **26.11.2019**

(51) International Patent Classification (IPC):
***C07D 403/04*** $^{(2006.01)}$ ***A61P 35/00*** $^{(2006.01)}$
***A61K 31/4196*** $^{(2006.01)}$

(52) Cooperative Patent Classification (CPC):
**C07D 403/04; A61P 35/00**

(86) International application number:
**PCT/TR2019/050998**

(87) International publication number:
**WO 2020/130974 (25.06.2020 Gazette 2020/26)**

(54) **NOVEL, NEW GENERATION INHIBITOR COMPOUNDS SPECIFIC TO GST P1 ENZYME**

NEUARTIGE INHIBITORVERBINDUNGEN DER NEUEN GENERATION, SPEZIFISCH FÜR DAS GST-P1-ENZYM

NOUVEAUX COMPOSÉS INHIBITEURS DE NOUVELLE GÉNÉRATION SPÉCIFIQUES DE L'ENZYME GST P1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2018 TR 201819936**

(43) Date of publication of application:
**19.05.2021 Bulletin 2021/20**

(73) Proprietor: **HACETTEPE ÜNIVERSITESI**
**06100 Altindag/Ankara (TR)**

(72) Inventors:
- **CEYHAN, Deniz**
  **Tekirdag (TR)**
- **SARIGÖL, Deniz**
  **16600 Gemlik/Bursa (TR)**
- **BÜBER, Esra**
  **Ankara (TR)**
- **UZGÖREN BARAN, Ay e**
  **06800 Çankaya/Ankara (TR)**

(74) Representative: **Yalçiner Patent and Consulting Ltd.**
**Tunus Caddesi 85/3-4**
**Kavaklidere**
**06680 Ankara (TR)**

(56) References cited:
**EP-A1- 1 988 098 WO-A1-2015/020553**

- **M. ABHILASH, S. UMA MAHESHWARI: "Insilico analysis of potential inhibitors for glutathione S-transferase of Wuchereria Bancroft causing lymphatic filariasis", RESEARCH & REVIEWS IN BIOSCIENCE, vol. 3, no. 1, 12 January 2009 (2009-01-12), pages 1-10, XP002803079, ISSN: 0974-7532**
- **SARIGOL, D. et al.: "Synthesis and structural studies of acyl hydrazone derivatives having tetrahydrocarbazole moiety", Journal of Molecular Structure, vol. 1086, 2015, pages 146-152, XP055721638,**
- **SHMEISS, N. A. M. M. et al.: "Synthesis of novel 1-substituted and 1, 9-disubstituted-1, 2, 3, 4-tetrahydro-9H-carbazole derivatives as potential anticancer agents", Molecules, vol. 5, no. 10, 2000, pages 1101-1112, XP055743934,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Technical Field of the Invention

The invention is related to the synthesis of novel and new generation inhibitor compounds specific to the GST P1 enzyme using computational drug design methods and uses thereof as anti-tumor agents in the pharmaceutical industry.

### Known State of the Art (Prior Art)

Drug resistance experienced during a cancer treatment process is one of the most important reasons that negatively affect the success of the treatment. Due to this reason, researchers are always carrying out studies to increase the efficiency of present drugs and to discovering new drugs that are more efficient.

One of the mechanisms that causes drug resistance in tumor cells is the interaction between Phase II conjugation enzymes and efflux transporter proteins that enable to expel agents such as drugs out of the cell. In this system particularly the expression of the glutathione S-transferase (GST) enzyme and "efflux" pumps in tumor cells is increased and depending on this, the activity of anticancer drugs is reduced (Tew 1994, Mukanganyama et al. 2002, Meijerman et al. 2008).

Glutathione S-transferase drugs are a member of the Phase II enzyme family which provides detoxification of endogenous and exogenous sourced, xenobiotic compounds (Tew 1994). The GST family is formed of three groups, being cytosolic, mitochondrial and microsomal (membrane bonded-MAPEG). The cytosolic GST's have been defined as 7 classes (alpha, mu, omega, pi, sigma, teta, zeta) until today depending on their amino acid sequence similarities, substrate specificity, and immunologic effects (Hayes et al. 2005, Mannervik et al. 2005).

The GST Pi (or GST P1) isozyme, is the most common GST form found in tumor cell sequences and neoplastic tissues (Sato 1989, Tew 1994, Mukanganyama et al. 2002). In almost all 60 tumor cells used in the anticancer drug screening program of the American National Cancer Institute GST P1 was determined as the dominant isozyme. The reduction of the susceptibility of tumor cells against anticancer agents has brought about drug resistance and particularly the excessive production of the P1 isoform of GST (Schultz et al. 1997, Morrow et al. 1998). Due to this reason, it is used as an important indicator of the clinical resistance that is developed against cytostatic drugs in drugs cancer drugs (Tidefelt et al. 1992, Jedlitschky et al. 1994, Mulder 1995, Armstrong 1997).

As the conjugates formed by GSH and several anticancer drugs are present in oxidative stress and xenobiotics; this brings to mind that these can be used as indicators of cellular stress. Indeed it has been determined that GSH-conjugates regulated the signal pathway activated with stress via GST isoenzymes (Adler et al. 1999).

The latest studies show that alpha, mu and pi isoenzymes play a regulatory role in protein kinase (MAPK) pathway that is activated with mitogen which plays a role in the cell viability and death signals (Burg and Mulder 2002).

The stress response of GST P1 which regulates the MAP kinase pathway via the protein: protein relation, acts as the endogenous inhibitor of c-Jun N-terminal kinase (JNK) which plays a role in apoptosis and cell proliferation (Wang et al. 2001, De Luca et al. 2012).

By means of the GST P1-JNK heterocomplex that is formed in cells that are not under stress, the apoptotic signal is suppressed. Additionally, apoptosis shall be induced by the separation of this complex under oxidative stress and by oligomerization of GST P1. The separation of said two proteins from each other also brings about different mechanisms (Townsend and Tew 2003) and the GST production that has increased in several tumor cells during drug treatment, changes the aftermath of the cell. It is difficult for the pressure on JNK to be removed due to increasing GST. Due to this reason, specific GST inhibitors have been developed in order to increase the cytotoxic effects of antineoplastic agents and to regulate cellular resistance against anticancer drugs. These drug candidates are included in four groups:

1. The compounds similar to glutathione (peptidomimetics), are known as competitive inhibitors of the GSH binding region (Cui et al. 2008). The aim is to increase isozyme selectability or to increase drug stability. The clinical studies of TLK 199 (or TER 199, γ-glutamyl-S-[benzyl]cysteinyl glycidyl diethyl ester) which can be given as an example from this group is being continued (Flatgaard et al. 1993, Cui et al. 2008).

2. Inhibitors having an electrophilic functional group which can form the GSH conjugate under *in vivo* conditions. The inhibitors in this group, pose a problem as they can react with GSH even when GST is not present (Ricci et al. 2005, Turella et al. 2005).

3. Inhibitor compounds that cause the formation of intermediary compounds which irreversibly inhibits GST activity (Zheng 2005).

4. The research for clinical applications of the prodrug TLK 286 (TER 286) which causes the release of toxin molecules via GST activation is being continued (Tew 2005).

The best characterized GST inhibitors until today are ethacrynic acid and glutathione analogue TLK 199. Ethacrynic acid binds with low affinity to the H region of the enzyme and is easily discharged out of the cell, as being conjugated with GSH. However in phase I studies, several problems have been encountered, such as diuresis, metabolic anomalies, and myelosupression (Schultz et al. 1997).

Besides these, non- GSH peptidomimetic derivatives of 7-nitro-2,1,3-benzoxadiazole (NBD) whose strong inhibitor effects have been observed for GST have been decided to be synthesized in order to avoid problems during inhibitor excretion from the cell via specific pumps. It is known that these NBD thioethers are bonded with high enzyme affinity to GST alpha, pi and mu isoenzymes (Ricci et al. 2005, Tew 2007). It has been shown that NBD's induced tumor cell death by starting apoptosis following the separation of the JNK-GST P1 complex (Tew 2007).

Although GSH derivatives are good inhibitors of GST's, as specific export pumps (MDR protein) are used to discharge these derivatives in order to avoid intracellular accumulation, they cannot show their effect on cancer cells. The GST P1 concentration in cancer cells reach up to $0.05\times10^{-3}$ M. The inhibitors to be used in equal or higher concentration in order to inhibit an enzyme at such a level shall also lead to undesired cytotoxicity in non-targeted cells. New molecules have been designed in order to overcome this problem, which can efficiently bind to GST, however which is not a GSH derivative, and which has lipophilic features that can easily pass through the cell membrane (Ricci et al. 2005).

A region having different topographies and substrate specificities that can interact with electrophilic, partially hydrophobic or hydrophilic substrates, defined as the H- region besides the G region (the region which binds the physiological substrate glutathione is available. Km values at micromolar levels or even nanomolar levels in GST alpha, pi and mu isoenzymes and compounds which show moderate affinity against said compounds are required (Caccuri et al. 1996, Ricci et al. 2005).

The GSH, GST levels inside the system which metabolizes drugs that are related with resistance against anticancer drugs is important. The role of GST during the conjugation of glutathione (GSH), which is the most commonly found thiole molecule in a cell, with endogenous and exogenous sourced electrophilic compounds have been shown in various studies carried out with alkylation agents such as melphalan, chlorambucil and cyclophosphamide (Tew et al. 1988, Damia and D'Incalci 1998). The increasing concentration of GST is related with the increase in the detoxification of alkylating agents. Indeed high GSH concentration and increased GST activity has been observed in cells that have developed resistance against alkylating agents (Tew 1994). Studies are available, which show that GSH synthesis inhibitors such as buthionine sulfoximine or GST inhibitors such as ethacrynic acid, increased the susceptibility of cancer cells against alkylating agents (Tew et al. 1988, Rotili et al. 2015).

## Brief Description of the Invention and its Aims

The invention is related a triazole derived compound having a tetrahydrocarbazole structure, inhibitor molecules that can be conjugated to GSH and are specific to GST P1, the synthesis of said molecule, and carrying out docking and enzyme kinetic studies.

The invention is related to designing, synthesis, molecular characterization of triazole derived compounds having a tetrahydrocarbazole (THC) structure as inhibitor molecules specific to GST P1 and to carrying out *in silico* computational and enzyme kinetic analysis studies.

## Figures

**Figure 1.** $IC_{50}$ dose(Log)-inhibition graph of the spectrophotometric enzyme activity of GST P1 at concentrations of the ctapm1 compound between 0-100 $\mu$M.

**Figure 2.** kinetic analysis results of ctapm1 compound with GST P1. A and C are the GSH and CDNB dependent Michaelis-Menten graphic of the ctapm1 compound and B and D are the GSH and CDNB dependent Lineweaver-Burk graphs of the ctapm1 compound.

**Figure 3.** The schematic view of the THC fragment, its load distribution and elemental features. 2-dimension schematic view (a) of "2,3,4,9-tetrahydro-1H-carbazole"in (shortly tetra hydro or THC) and the load distribution (b) of the THC fragment. Elemental features of the THC fragment (scaffold): Mass = 171,2383; Exact mass = 171,104799421; Formula = $C_{12}H_{13}N$; Isotope formula = $C_{12}H_{13}N$; Dot-disconnected formula = $C_{12}H_{13}N$; Dot-disconnected isotope formula = $C_{12}H_{13}N$; Composition = C (84,17%), H (7,65%), N (8,18%); Isotope composition = C (84,17%), H (7,65%), N (8,18%); Atom count = 26.

**Figure 4.** Molecular interaction of the THC fragment with GST P1. a: 3-dimensional placement of the protein in the fragment, b: 2-dimension graphic view of the interaction of the fragment with protein residues.

**Figure 5.** molecular protein-ligand interactions of the *"scaffold"* of mTHC with GST P1 during a docking process.

**Figure 6.** The placements of THC and mTHC *"scaffolds"* together in the GST P1 catalytic region. THC was represented with green and mTHC was represented with gray.

**Figure 7.** protein-ligand interactions according to ctapm1-GST P1 *docking* calculations.

**Figure 8.** view of the 95 poses together that are located in the same cluster with 2A° RMSD tolerance during ctapm1-GST P1 *docking* calculations.

**Figure 9.** molecular *"docking"* of the mtas fragment and GST P1 protein.

**A.** Interaction respectively 3B and 2B, of the type-2 position of mtas fragment with protein residues.
**B.** Collective presentation of the independent docking calculation results of ctapm1 compound and mtas fragment. The carbon atoms of the compound ctapm1 have been presented as gray and the red compound is mtas fragment.

**Detailed Description of the Invention**

The compounds were synthesized and the purity of their structure was confirmed by Infrared (IR), UV, Nuclear Magnetic Resonance (NMR) and Mass spectroscopy and elemental analysis. Not only is the compound novel, but also it has GST P1 enzyme inhibiting activity and the determination of the values belonging to this has been carried out for the first time in this study. Empirically proving the theoretically estimated structures together with synthesizing the compounds, constitutes the most significant part of this study. The synthesized compounds are compounds that are not present in literature and they are synthesized for the first time. Therefore, the synthesis method is also novel.

1-acyl thiosemicarbazide compound has been synthesized as a result of reacting the tetrahydrocarbazole compound with thiosemicarbazide compound. Triazole compound having a tetrahydrocarbazole structure is synthesized as a result of refluxing 1-acil thiosemicarbazide compound in NaOH solution, under a condenser.

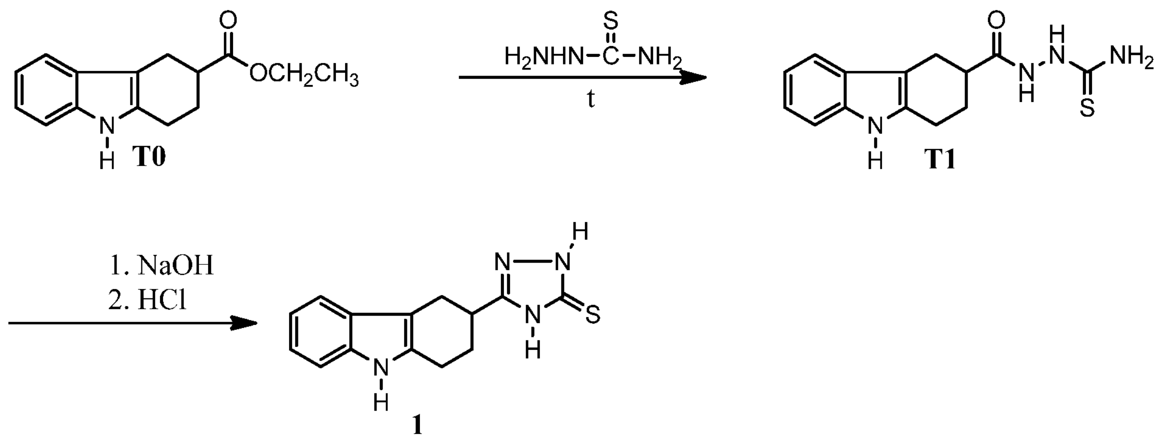

T0
T1
1

**Scheme 1.** Synthesis plan of the triazole compound.

The characterization of the synthesized compounds has been conducted using NMR spectroscopy method.

2-(2,3,4,9-tetrahydro-1H-carbazole-3-carbonyl)hydrazinecarbothioamide (**T1**)
E.N.: 132°C - degradation
Yield=62%
$^1$H NMR (DMSO-$d_6$, 400 MHz) δ: 1.79-1.83 (m, 1H, CH), 2.14-2.17 (m, 1H, CH), 2.50-2.78 (m, 4H, 2$CH_2$), 2.81-2.92 (m, 1H, CH), 6.91-7.01 (m, 2H, 2ArH), 7.24 (d, 1H, J=7.87 Hz, ArH), 7.33 (d, 1H, J= 7.60 Hz, ArH), 7.43 (s, 1H, NH), 7.89 (s, 1H, NH), 9.22 (s, 1H, NH), 9.85 (s, 1H, NH), 10.68 (s, 1H, NH)

**Method 1**

**3-(2,3,4,9-tetrahydro-1H-carbazole-3-vl)-1H-1,2,4-triazole-5(4H)-thione (1)** (Has been shortly referred to as ctamp1 in the text and the figures).

0,007 mol (2,0 g) 2-(2,3,4,9-tetrahydro-1H-carbazole-3- carbonyl) hydrazinecarbothioamide (T1) compound has been refluxed for 3 hours with 10% 60ml KOH and has been filtered. The obtained filtrate is acidified with diluted HCl. The obtained residue is filtered and is washed with water and dried. The structure of the synthesized 3-(2,3,4,9-tetrahydro-1H-carbazole-3-yl)-1H-1,2,4-triazol-5(4H)-thione (1) compound is verified with $^1$H-NMR spectrum.

E.N.: 255°C - degradation
Yield=90%
$^1$H NMR (DMSO-$d_6$, 400 MHz) δ: 2.04-2.12 (m, 1H, CH), 2.27-2.30 (m, 1H, CH), 2.78-2.96 (m, 3H), 3.06-3.15 (m, 2H), 6.98-7.08 (m, 2H, 2ArH), 7.31 (d, 1H, J=7.86 Hz, ArH), 7.41 (d, 1H, J= 7.67 Hz, ArH), 10.80 (s, 1H, NH), 13.32 (s, 1H, NH), 13.34 (s, 1H, NH).

Upon obtaining positive results after the completion of the studies on the compound numbered **1**, that was synthesized, the core structure of this compound was preserved and derived compounds were determined and the interactions of these compounds with hGST P1, have been examined with AutoDock4.2 program (AD) in detail theoretically. The results for these compounds have been provided in Table 1.

**Synthesis methods:**

The synthesis methods for the compounds provided in Table 1 have been listed below.

**Method 2: Synthesis method for triazole compounds having a tetrahydrocarbazole structure**

Tetrahydrocarbazole compounds, were synthesized with the Fischer Indol method, which is one of the basic synthesis methods. Mixture of 1.1 mol phenylhidrazinium hydrochloride derivative and 1 mol 4-carboethoxy cyclohexanone in ethanol were refluxed under nitrogen atmosphere for 8 hours. The reaction mixture was cooled to room temperature and the solvent is removed. The remaining is dissolved in chloroform and is first of all extracted with 10% HCl acid and then with 10% $Na_2CO_3$. The organic phase is dried. The solvent is removed. Crystallization is performed from the suitable solvent. The processes mentioned in Method 1 are applied to the these newly synthesized tetrahydrocarbazole derivatives to get triazole compounds having -Br and -F bonded to the aromatic ring of the tetrahydrocarbazole structure.

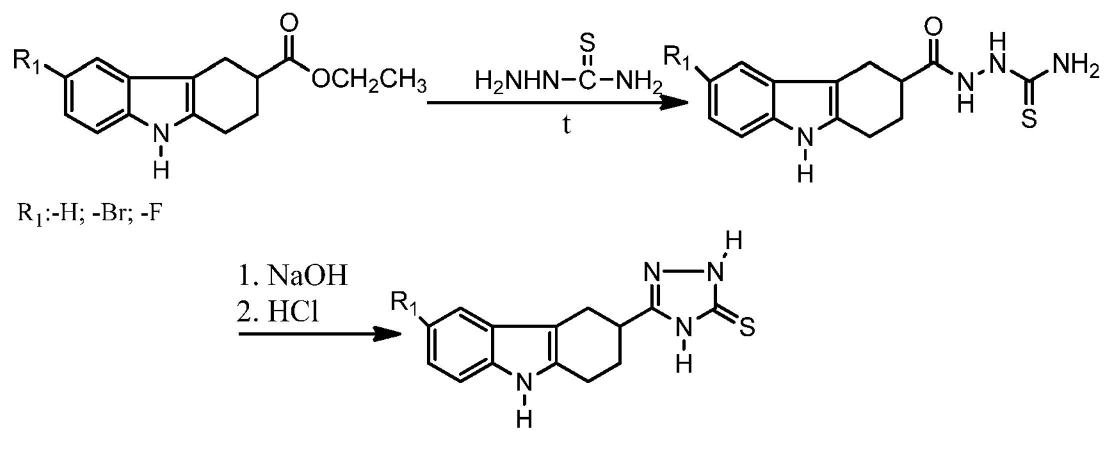


**Scheme 2.** Synthesis plan for Compound 1, 19 and 23

**Method 3: Synthesis method for triazole compounds having an N-Methyl tetrahydrocarbazole structure**

1.0 mol tetrahydrocarbazole derivative **(T0)** that is dissolved in dichloromethane, is cooled to 0 °C. After this 50% sodium hydroxide having the same ratio as the solvent, catalytic amounts of tetrabutylammonium hydrogen sulphate and 1.5 mol methyl iodine is added inside. The mixture is kept for 1 hour at 0 °C temperature and then stirred overnight

for 1 night at room temperature. It is then extracted with 10% HCl. The organic phase is dried. The solvent is removed. Crystallization is performed from the suitable solvent. The processes mentioned in Method 1 are applied to the synthesized N-methyl tetrahydrocarbazole compounds and -H and -F triazole derivatives bonded to the aromatic ring of the tetrahydrocarbazole structure are synthesized.

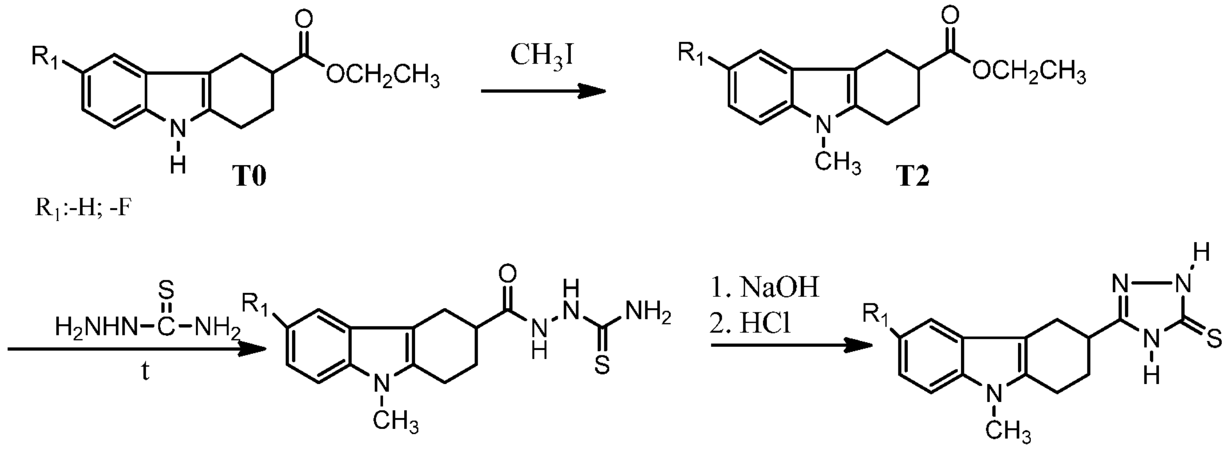


**Scheme 3.** Synthesis plan for Compound 24 and 25

**Method 4: Synthesis method for triazole compounds having a carbazole structure**

22.5 mol of 2,3-Dichloro-5,6-dicyano-1,4-benzoquinone (DDQ) is added into the 1.0 mol tetrahydrocarbazole derivative that was dissolved in tetrahydrofuran. The reaction is refluxed for 24 hours. The solvent is removed. The solid matter is dissolved with ethyl acetate and is extracted with 10% $Na_2CO_3$. The organic phase is dried. The solvent is removed. Crystallization is performed from the suitable solvent. Triazole derivatives are synthesized having a novel carbazole structure, by applying the processes provided in Method 1 to the synthesized carbazole compounds.

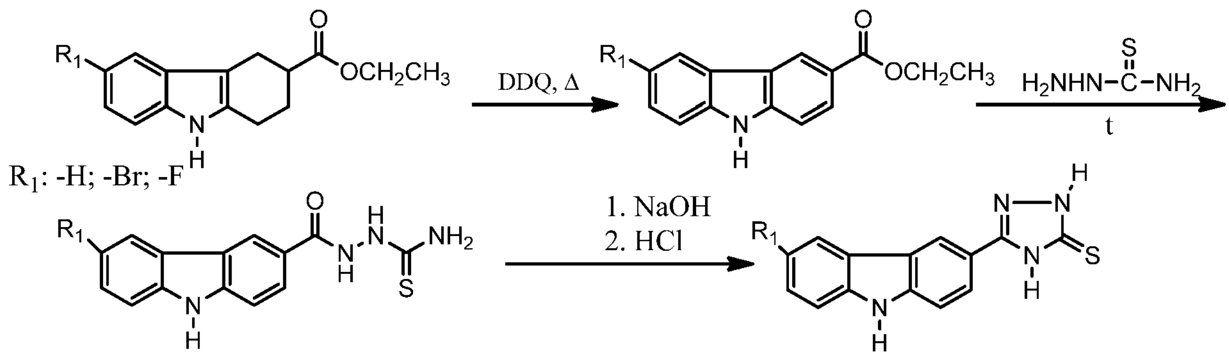


**Scheme 4.** Synthesis plan for Compound 9, 11 and 13

**Method 5: Synthesis method for triazole compounds having a N-Methyl carbazole structure**

2.5 mol DDQ compound is added into the 1.0 mol N-methyl tetrahydrocarbazole derivative that was dissolved in tetrahydrofuran. The reaction is refluxed for 24 hours. The solvent is removed. The solid matter is dissolved with ethyl acetate and is extracted with 10% $Na_2CO_3$. The organic phase is dried. The solvent is removed. Crystallization is performed from the suitable solvent. Triazole derivatives are synthesized having a novel N-methyl carbazole structure, by applying the processes provided in Method 1 to the synthesized carbazole compounds.

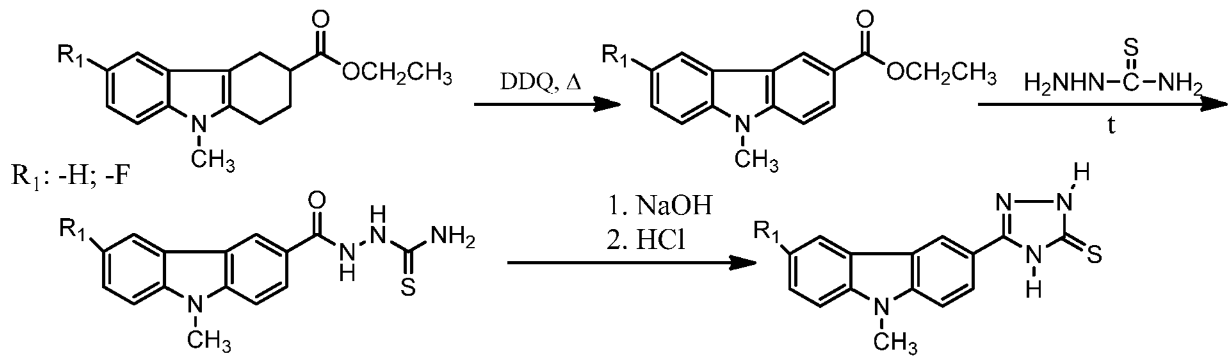


**Scheme 5.** Synthesis plan for Compound 5 and 6

**Method 6: Synthesis method for triazole compounds having a 1-Oxo tetrahydrocarbazole structure**

2 mol periodic acid is dissolved in 1:2 water : methanol mixture and is cooled to 0 °C. After this 1 mol triazole derivative dissolved in THF is added slowly. The mixture is stirred for 4 hours. The solvent is removed following the reaction. The remaining part is dissolved in chloroform. It is then extracted with 10% $Na_2CO_3$ and 10% $NaHSO_3$. The organic phase is dried. The solvent is removed. The triazole compounds that are obtained, having 1-oxo-tetrahydrocarbazole structure is crystallized from cyclohexane (Scheme 6).

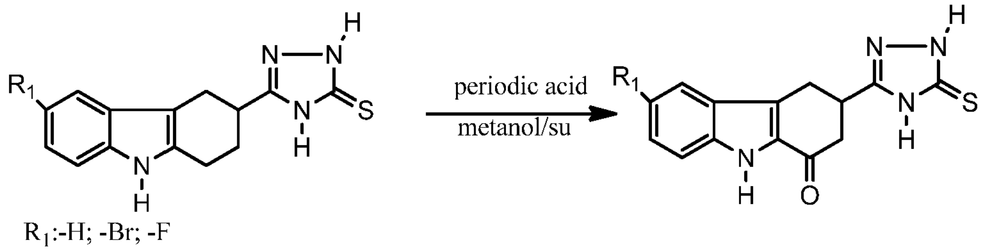


**Scheme 6.** Synthesis plan for Compound 4,7 and 8

**Method 7: Synthesis method for triazole compounds having a N-Methyl-1-oxo tetrahydrocarbazole structure**

2 mol periodic acid is dissolved in 1:2 water : methanol mixture and is cooled to 0 °C. After this 1 mol N-methyl triazole derivative dissolved in THF is added slowly. The mixture is stirred for 4 hours. The solvent is removed following the reaction. The remaining part is dissolved in chloroform. It is then extracted with 10% $Na_2CO_3$ and 10% $NaHSO_3$. The organic phase is dried. The solvent is removed. The triazole compounds that are obtained, having N-methyl-1-oxo-tetrahydrocarbazole structure is crystallized from cyclohexane (Scheme 7).

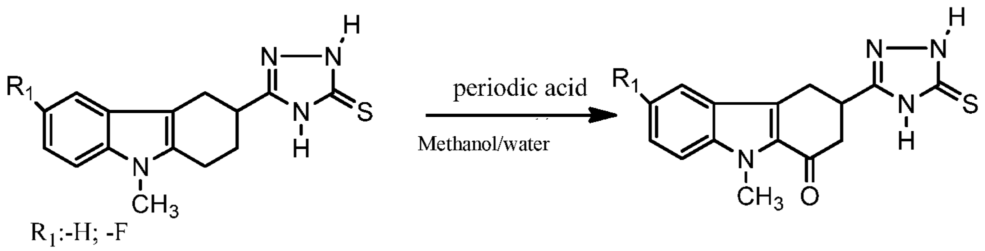


**Scheme 7.** Synthesis plan for Compound 2 and 3

**Method 8: Synthesis method for triazole compounds having a 4-Oxo tetrahydrocarbazole structure**

1 mol triazole derivative is dissolved in tetrahydrofuran (90%). 2 mol DDQ that is dissolved in tetrahydrofuran

is added at 0 °C. The reaction is stirred at room temperature for 12 hours. The mixture is poured into 5% potassium carbonate. It is then extracted with ethyl acetate. The organic phase is dried. The triazole compounds that are obtained, having 4-oxo-tetrahydrocarbazole structure is crystallized from ether (Scheme 8).

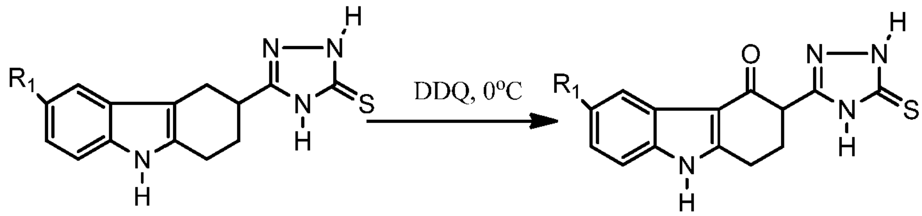


$R_1$:-H; -Br; -F

**Scheme 8.** Synthesis plan for Compound 14, 16 and 18

**Method 9: Synthesis method for triazole compounds having a N-Methyl-4-oxo tetrahydrocarbazole structure**

1 mol N-methyl triazole derivative is dissolved in tetrahydrofuran (90%). 2 mol DDQ that is dissolved in tetrahydrofuran is added at 0°C. The reaction is stirred at room temperature for 12 hours. The mixture is poured into 5% potassium carbonate. It is then extracted with ethyl acetate. The organic phase is dried. The triazole compounds that are obtained, having N-methyl- 4-oxo-tetrahydrocarbazole structure is crystallized from ether (Scheme 9).

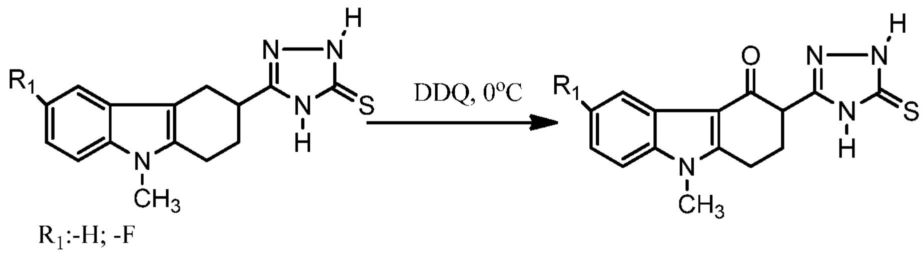


$R_1$:-H; -F

**Scheme 9.** Synthesis plan for Compound 10 and 12

**Method 10: Synthesis method for triazole compounds having a 1, 4-dioxo tetrahydrocarbazole structure**

Triazole derivative having a 1 mol 1-oxo-tetrahydrocarbazole structure is dissolved inside tetrahydrofuran (90%). 2 mol DDQ that is dissolved in tetrahydrofuran is added at 0°C. The reaction was stirred for 12 hours at room temperature. The mixture is poured into 5% potassium carbonate. It is then extracted with ethyl acetate. The organic phase is dried. It is then crystallized from ether (Scheme 10).

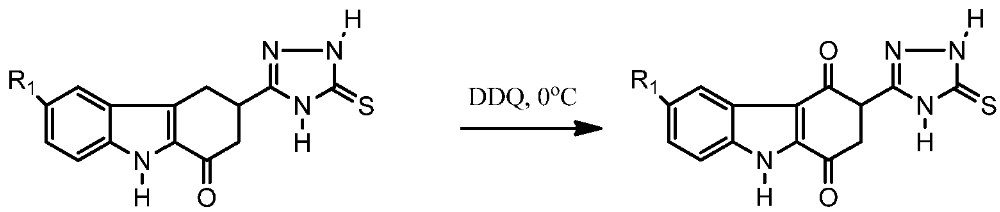


$R_1$:-H; -Br; -F

**Scheme 10.** Synthesis plan for Compound 15, 21 and 22

**Method 11: Synthesis method for N-methyl-1,4-dioxo triazole compounds having a tetrahydrocarbazole structure**

Triazole derivative having a 1 mol N-methyl 1-oxo-tetrahydrocarbazole structure is dissolved in tetrahydrofuran

(90%). 2 mol DDQ that is dissolved in tetrahydrofuran is added at 0 °C. The reaction was stirred for 12 hours at room temperature. The mixture is poured into 5 % potassium carbonate. It is then extracted with ethyl acetate. The organic phase is dried. It is then crystallized from ether (Scheme 11).

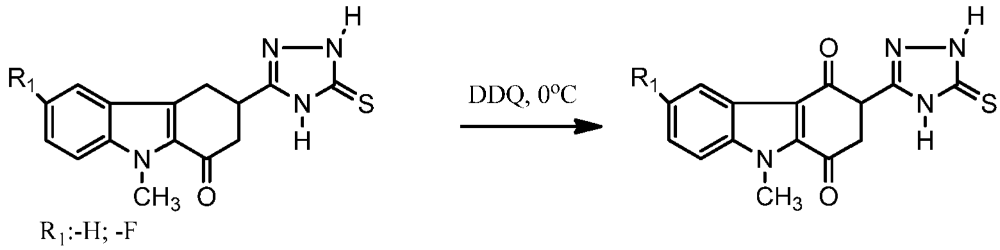


**Scheme 11.** Synthesis plan for Compound 17 and 20

### Enzymatic Inhibition Tests

Pure human-GST P1 (hGST P1) enzyme has been used as target protein in enzyme inhibition tests. "Ethacrynic acid" has been used as the model inhibitor compound. Ctapm1 (compound 1) compound which is a tetrahydrocarbazole derivative has been used as the basic precursor inhibitor for compounds that are designed, synthesized and are planned to be synthesized with advanced modifications.

### Spectrophotometric Enzyme Activity

The enzyme activity was determined over the activity of GST P1 enzyme which conjugated GSH and 1-chloro-2,4-dinitrobenzene (CDNB). The test medium was prepared to include 100 mM sodium phosphate buffer (pH 6.5) comprising 1 mM EDTA, 1 mM GSH, 1 mM CDNB and 5% ethanol and the temperature of the medium was determined as 30°C. The formation of a conjugate (GS-DNB) was monitored for 1 minute over the absorbance increase at 340 nm wavelength. The specific activity was calculated using 0.0096 $\mu M^{-1}$ $cm^{-1}$ absorption coefficient. The enzymatic reaction was carried out with 1 mL volume, at plastic spectrophotometry forces where the light path was 1cm. The reaction was started by adding CDNB. The specific activity calculation was provided as "$\mu$mol.minute$^{-1}$.mg$^{-1}$" by the conversions given in Formula 1.

$$\text{Specific Activity } (\mu\text{mol x minute}^{-1} \text{ x mg}^{-1}) = \frac{\Delta A \times V_r \times d}{\varepsilon \times \ell \times (\text{enzyme}) \times V_e} \qquad \textbf{(Formula 1)}$$

$\Delta A$: Absorbance change ($A_{340}$/minute.ml)
$V_r$: Reaction volume (ml)
d: Dilution factor
$\varepsilon$: Molar absorption coefficient (9,6 $mM^{-1}$, $cm^{-1}$)
$\ell$: Light path (0,524 cm for 1 cm microplate measurements for Standard spectrophotometry vessel,
$V_e$: Volume added to the stock enzyme reaction medium (ml)

### Activity Measurement on the Microplate

The activity measurement was performed in 96-well microplates where, other components had constant concentrations and where, in each measurement series, each one of the CDNB, GSH and candidate inhibitor molecules may have had variable concentrations. The total reaction was carried out in 200 $\mu$L together with an activity buffer. After the constant concentration enzyme in pH 6.5 100 nM potassium phosphate buffer was incubated at certain periods of time with different concentrations of the candidate inhibitor, the reaction was added to the medium where the final concentration of GSH in the same buffer was 1 mM, and it was incubated for 3 minutes. As a result of this incubation, CDNB that was prepared in ethanol whose final concentration was 5% ethanol and 1 mM CDNB was added and a reaction was started. The reaction was tracked in spectrophotometry for 5 minutes at 340 nm. The spectrophotometric measurement was carried out with "Molecular Devices, SpectraMax M2" and the measured data was evaluated with "Basic Kinetic Protocol" in a "SoftMax Pro 4.8" program. The specific activity was calculated according to Formula 1, but during the calculation the light path was used as 0,552.

### Determination of $IC_{50}$ values of the Compounds during Enzyme Inhibition

The $IC_{50}$ value is defined as the concentration value where the inhibitor compound inhibits 50% of the enzyme activity. In order to determine $IC_{50}$ values of compounds both the standard spectrophotometric method and the microplate kinetic reading method was used together. 10 mM stock solutions of compounds in dimethyl sulphoxide (DMSO) were prepared. The final concentrations at the reaction medium were tried to be maintained between 0-250 μM. Kinetic reaction was started by adding CDNB at the end of the 10 minute incubation with enzyme, for the series formed of at least 6 points at concentration of the compounds that were variable between 0-250μM. The reaction readings in spectrophotometry were tracked for 2 minutes in 1 mL plastic tanks with a standard method, and for 5 minutes in a microplate.

The enzymatic reaction series where an inhibitor was not used was accepted as control. The control, was taken as 100% as a result of the reaction and the percentage conversions of other inhibitor-enzyme reaction results were carried out. The compound concentration where 50% inhibition was achieved as evaluated as $IC_{50}$.

The statistical examinations of enzymatic kinetic assay data and the drawing of graphics was carried out with a GraphPad Prism program.

### Activity Tests of the Compound

As a result of the theoretical studies mentioned above, the synthesis of the ctapm1 compound was carried out and activity tests were performed with GST P1. When the $IC_{50}$ dose(Log)-inhibition graph of the GST P1 spectrophotometric enzyme activity was evaluated at concentrations between 0-100 μM of the ctapm1 compound, it was found that the ctapm1 compound, inhibited GST P1 activity with 1,07 μM $IC_{50}$ value (According to GraphPrism program calculations) (**Figure 1**).

It was found that the $K_i$ inhibition constants of the ctapm1 compound against GSH was 1,735 μM at non-competitive type and against CDNB was 1,701 μM at competitive type. (**Figure** 2).

### Computer based Design and Docking Analysis Examinations of ctapm1 Compound: Standardizing Docking protocol with AutoDock.

In order to be able to use scoring function results comparatively, a complete docking protocol standardization was carried out for each of the *docking* tests.

The procedures that have been carried out during the study were conducted as briefly given below:
For the protein model to be used during docking calculations, the crystal structure solutions in the Protein Data Bank have been used (Berman et al. 2000). By using 3GSS, 2GSS, 11GS and 18GS pdb code structures, a three dimensional hybrid crystal model structure was obtained for the hGST P1 protein. All of these models were superimposed to obtain this and protein chains were aligned, and then the necessary structures were extracted and a hybrid 3B hybrid crystal hGST P1 structure was prepared. Protein chains, separated and protonated GSH (glutathione) and EAA structures (ethacrynic acid) were obtained from 3GSS; a binding form to EAA at the absence of GSH was obtained from 2GSS; GS-EAA conjugate was obtained at the presence of GSH from 11GS and the GSH and EAA bonding structures were cut off and proton was added; CDNB and GSH was obtained separately from 18GS; GSH and NBDHEX (6-nitro-2,1,3-benzoxadiazole-4-ylthio hexanole) molecules were obtained from 3GUS. By using these extracted structures, the GSH binding region on the protein and the model substrate and/or inhibitor's binding regions were determined.

- This model structure was added into AutoDock Tools (ADT) and following this all of the nonpolar hydrogen's were removed and their loads were added to protein. Water and other non protein molecules were removed from the structure.

- "Grid.gpf" AutoGrid parameter file was prepared with ADT.

    ◦ The grid box (3 dimensional area) was centered to the H-region where the ethacrynic acid at the active region of the B sub unit of the homodimer macromolecule and the above mentioned other substrate was located.

    ◦ The number of the grid position was adjusted to be 28 for every direction and the interposition range was determined to be 0.375 A°. By this means the gridbox area was configured to be large such that the ligand could rotate inside it comfortably.

- Dock.dpf AutoDock parameter file was prepared with ADT.

    ◦ "Search Algorithm" has been selected as "Lamarckian Genetic Algorithm".

◦ Genetic algorithm settings: Runs: To be resulted with 150, 150 possible conformation; Population size: 50 individuals; Number of energy evaluation: as 1,500,000, *Docking* parameters: *Translation:* Has been arranged as 0.2 A°; *Quaternation*: 5.0°; *Torsion:* 0.5 °; *RMS cluster tolerance:* 0.75 A°

- For the validation of the AutoDock 4.2 program EAA was extracted from the gstp_hib model and a *docking* process was carried out again with the above mentioned parameters. Examination was carried out according to the original structured EAA position and conformation of the *redocked* EAA. The *docking* procedure and parameters were standardized until the *Root Mean Square Deviation* (RMSD) was found to be less than 2 A°.

- Each ligand was placed in ADT and afterwards, in order to calculate loads, first of all it was enabled for all hidrogens to be added to the ligand-mol2 structure and it was prepared for *docking.* When de ligand was loaded, ADT automatically added Gasteiger loads to the ligand atoms, it was then determined which bonds could be twisted/turned and all nonpolar hydrogens were removed. Nonpolar H-atoms were erased and the loads were added to C-atoms.

- When preparing all ligand libraries in order to compare the *docking* scores of molecules obtained with design and virtual scanning libraries with the *docking* score of the model inhibitor molecule EAA, it was ensured that all docking conditions such as energy minimization-conformation, addition of hydrogen atoms were the same. If it was deemed necessary, molecular descriptive files in SMILES format of all the other ligand libraries together with the model molecule was established; following this, these were again converted to molecular descriptive files in 3B-mol2 format with a suitable program.

- Examination of AutoDock Results: The results were analyzed with the AutoDock Tool (ADT) by the aid of "Grid_data_file", "atom specific map files," electrostatic.map" files created during the *docking* process and by using a ".dig" file which is a *docking* process file. Visual analyses and the analysis of protein-ligand interactions were performed with Discovery Studio 3.5 program.

### ***Docking* Analysis Findings**

### ***Scaffold* fragment tetrahydrocarbazole (THC) *Docking* Examination**

It was believed by our group that the THC fragment could be a suitable *scaffold* in advanced GST P1 inhibitor designs according to the findings of prior studies carried out with compounds having a tetrahydrocarbazole core structure. *Docking* examinations were carried out in relation to this fundamental molecular structure. Some elemental features regarding the molecule structure and the load distribution of the molecule was determined with MarvinBeans (Marvin 6.0.1, ChemAxon http://www.chemaxon.com) (**Figure 3**).

Docking calculations of the THC *scaffold* (fragment) with GST P1 was carried out and the THC-GST P1 molecule interactions were examined (**Figure 4**).

During docking calculations it was noted that the *"scaffold"* was stably positioned in the hydrophobic pocket of the enzyme catalytic region (H-site). It was noted that Tyr108 and pi-pi, Tyr7 and H-bond interactions were significant in the stabilization of the molecule. It was also observed that van der Waals interactions with Ile104, which were descriptive residues of allelic variations of Gly12, Gly205, Val10, Phe8 and GST P1 and electrostatic interactions with Tyr7, Arg13 and cofactor GSH was established. (**Figure 4**).

During the clustering of the 100 poses having the best score obtained according to AD4 calculations with RMSD ≤ 2A° tolerance, it was observed that 60 poses were in one cluster and 40 poses were in the other cluster, thereby forming two large clusters. The average docking score of the Cluster -1 poses, was determined as -5.87 kcal/mol at 25 °C and the calculated $K_i$ constant was determined as 49.83 μM. The ligand efficiency (LE) value was calculated as 0.45. The average binding affinity of Cluster -2, was determined as -5.86 kcal/mol and the calculated $K_i$ constant was determined as 50.63 μM.. In group 1 where the scaffold docking poses are clustered, the cyclohexane ring in the scaffold structure is directed into the H-ligand binding pocket of the target protein, while the benzene ring in the scaffold structure is directed out of the H-ligand binding pocket of the target protein. It was noted that Cluster-2 poses were at the same position of as the Cluster 1 poses; however that they were positioned exactly 180 degrees in reverse (Visual presentation has not been provided).

### **Modification of the "cyclohexane" section of the *"Scaffold"***

It was observed that placement of the *"cyclohexane"* section of the molecule of THC at the H-region of the protein during repeated docking examinations was directed nearly always either into the hydrophobic region at the inner section of the H-pocket or outside the H-pocket at a 180 degree reverse position. A methyl group was added to the C12

atom in order to increase the stability of the *"Scaffold"* in this region and a docking examination was repeated. The THC molecule that has been modified has been abbreviated as mTHC in this text.

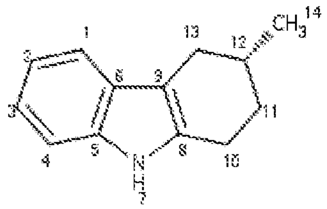

**Scheme 12.** THC structure modified with a methyl group in C12 carbon. Suggested IUPAC name: (3R)- 3-methyl- 2,3,4,9- tetrahydro- 1H-carbazole.

During the *docking* calculations of the tetrahydrocarbazole *"scaffold"* that is modified with the methyl group, pose positioning similar to Group-2 was observed which were similar to THC docking results. Together with this the mTHC fragment presented a more stable interaction with GST P1. It has been observed that while the THC docking comprises 40 of the 100 docking poses with Group-2 (or Cluster-2) 2°A RMSD tolerance, mTHC *docking* comprises 98 of the 100 *docking* poses with RSMD tolerance. Therefore it was unavoidable for both compounds to interact the same way with protein residues. As an important finding, it was noted that by the addition of a methyl group to the C12 atom of cyclohexane the stabilization of similar positioning of the *"scaffold"* inside the protein increased from 40% to 98%. The average binding affinity of the mTHC structure was determined as -6.12 kcal/mol and the calculated $K_i$ constant was determined as 32.78 μM **(Figure 5).**

When the docking poses of THC and mTHC compounds were examined together, it was seen that the poses overlapped in almost exactly the same coordinates except for a very small separation in cyclohexane sections, however it was observed that the mTHC fragment interacted more stably with GST P1 in comparison to THC. By this means, it has been decided that THC could significantly affect the binding stability if it was modified in any way by C12 (**Figure 6**).

**Docking Analysis findings of the Suggested Compound ctapm1 (*3-(2,3,4,9-tetrahydro-1H-carbazole-3-yl)-1H-1,2,4-triazole-5(4H)-thione (compound: 1)*)**

The theoretical examinations on THC and mTHC structures have led the way for advanced chemical modifications to be carried out these scaffolds. As a result, ctapm1, was determined as a compound which is TCH-derivative compound comprising a triazolthione functional structure. Some features related to the molecule structure have been determined by MarvinBeans (ChemAxon) and this has been given in **Scheme 13.**

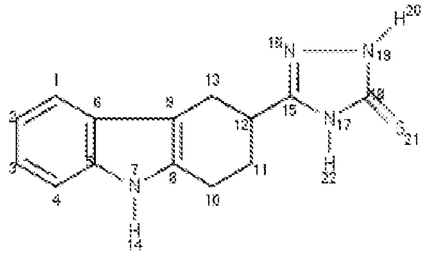

**Scheme 13.** Suggested TCH-derivative novel compound: ctapm1

**Suggested IUPAC name: 3-(2,3,4,9-tetrahydro-1H-carbazole-3-yl)-1H-1,2,4-triazole-5(4H)-thione**; Elemental Features (Calculated with MarvinBeans): Mass = 270,353; Exact mass = 270,093917158; Formula = $C_{14H14N4}S$; Isotope formula = $C_{14H14N4}S$; Dot-disconnected formula = $C_{14H14N4}S$; Dot-disconnected isotope formula = $C_{14H14N4}S$; Composition = C (62,2%), H (5,22%), N (20,72%); Isotope composition = C (62,2%), H (5,22%), N (20,72%); Atom count = 33.

Docking calculations of ctapm1 and GST P1 have been repeated several times. In nearly all of the docking process data, similar *docking* poses and binding affinity scores were obtained. It was noted that the binding affinities for the ligand placements collected in the same cluster ranged quite homogenously from -7.60 to -7.74 kcal/mol, while the $K_i$ constants again homogenously ranged from 2.13 to 2.67 μM.

It was noted that the carbazole portion of the ctapm1 compound was located at the hydrophobic region in the H-pocket of the protein catalytic region, while the triazolthione portion interacted with relatively more hydrophilic sections. It was observed that in independent docking applications and in the ligand-protein placement poses obtained during docking applications, were formed almost entirely in the same coordinates **(Figure 7)**. According to ctapm1-GST P1 *docking* calculations, it was found that in protein-ligand interactions, and in particular the triazolthione portion of the compound overlapped each other at the same coordinates, such that different poses could not be distinguished from each other **(Figure 8)**. In this stabilization, it was determined that the electrostatic interaction with Arg13, the electrostatic and H-bond interactions with Tyr7, and the cation-pi interactions with Tyr108 and H-bond of the triazolthione portion of the compound, were significant. Additionally the close electrostatic interaction of GSH with N16 atom was also found to be remarkable (**Figure 7 and 8**).

The low torsional free energy of ctapm1 compound in this natural placement in the GST P1 catalytic region was found to be an important factor. Due to this reason, the "3-methyl-4,5-dihydro-1H-1,2,4-triazolthione-5-thione" (this has been abbreviated here as "methyltriacolthione" or "mtas") portion of the ctapm1 compound was extracted and docking procedures were carried out at the same catalytic region of GST P1. Some physicochemical features of this fragment that has been calculated with the ChemAxon program has been presented in **Scheme 14.**

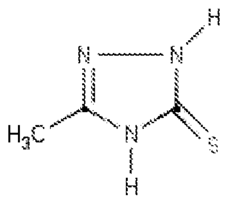

**Scheme 14.** Some physicochemical features of the methyltriazolthione fragment.

Suggested IUPAC name: 3-methyl-4,5-dihydro-1H-1,2,4-triazolethione-5-thione; Elemental Features: Mass = 115,157; Exact mass = 115,020417865; Formula = $C_3H_5N_3S$; Isotope formula = $C_3H_5N_3S$; Dot-disconnected formula = $C_3H_5N_3S$; Dot-disconnected isotope formula = $C_3H_5N_3S$; Composition = C (31,29%), H (4,38%), N (36,49%), S (27,84%); Isotope composition = C(31,29%), H (4,38%), N (36,49%), S(27,84%); Atom count = 12, LogP: 0.3.

It has been found that during *Docking* procedures the **mtas** fragment shows only two types of settlements at the protein catalytic region (Type-1 and Type-2). The ligand bonding affinity average in these two clusters have been determined as -4.45 ve -4.65 kcal/mol. It was found that mtas-GST P1 docking settlements were quite homogeneous and were carried out at high frequency.

It was observed that mtas fragment had more natural interactions with the protein residues of type-2 which is one of the clusters in GST P1 protein. Particularly the same interaction established by **ctapm1** compound, with the protein residues of the triazolthione group were also observed in the type-2 placements of the mtas fragment **(Figure 9).**

As a result of the docking examinations mentioned above, it was determined that natural and high affinity interactions could be carried out by the **ctapm1** compound in the determined GST P1 catalytic region. Especially, it was noted that methyl triazolthione group was not only included in the compound structure but it was also positioned the same way on the protein, following the docking examinations that were conducted **(Figure 9).**

These results have set forth with empirical results , at a molecular level and in a conclusive manner that the ctapm1 compound was a high affinity and specific ligand for GST P1.

In accordance with the above mentioned findings, novel GST P1-specific inhibitors that have high synthesizable potentials have been designed **(Table 1).** The interactions of these compounds with hGST P1, have been examined with AutoDock4.2 program (AD) in detail theoretically and the results were given in **Table 1**. The binding affinities of the compounds to hGST P1 have been given with kcal/mol values, and the inhibition constants have been given as (Ki) μM.

**Table 1.**

| | Compound | CdId | AD, kcal/mol | Ki, μM |
|---|---|---|---|---|
| 1 | 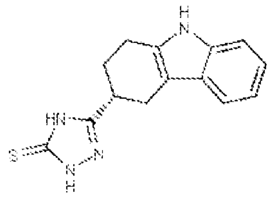 | 1 | -7.74 | 2.13 |
| 2 | 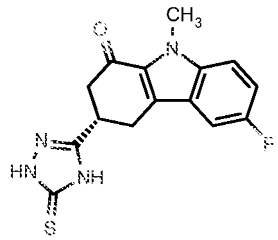 | 1021 | -8.14 | 1.08 |
| 3 | 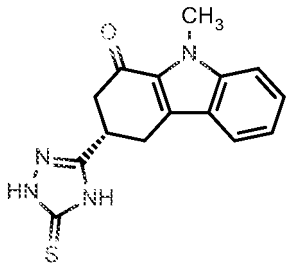 | 1020 | -8.12 | 1.11 |
| 4 | 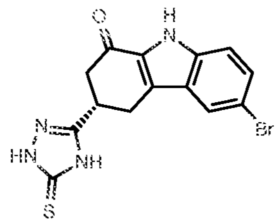 | 1024 | -7.86 | 1.74 |
| 5 | 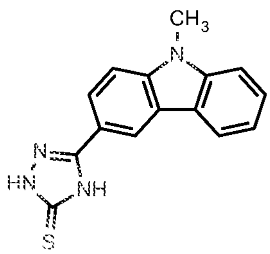 | 1004 | -7.82 | 1.86 |
| 6 | 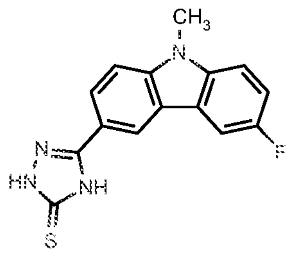 | 1005 | -7.66 | 2.41 |

(continued)

| | Compound | CdId | AD, kcal/mol | Ki, μM |
|---|---|---|---|---|
| 7 | 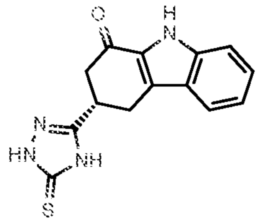 | 1022 | -7.65 | 2.47 |
| 8 | 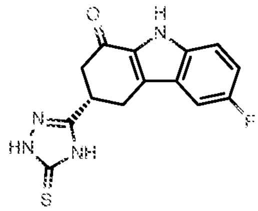 | 1023 | -7.63 | 2.57 |
| 9 | 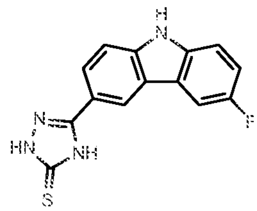 | 1002 | -7.14 | 5.82 |
| 10 | 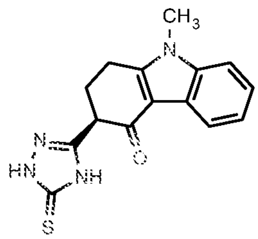 | 1016 | -7.12 | 6.08 |
| 11 | 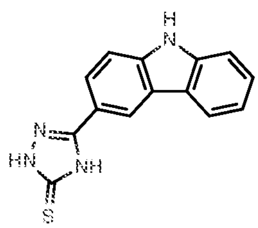 | 1001 | -7.12 | 6.03 |
| 12 | 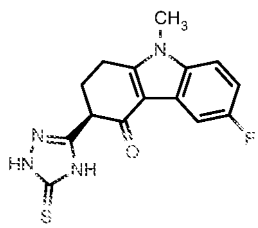 | 1017 | -7.05 | 6.81 |
| 13 | 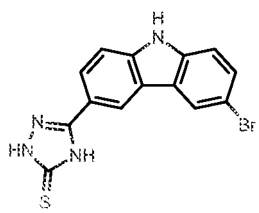 | 1003 | -7,38 | 3.89 |

(continued)

| | Compound | CdId | AD, kcal/mol | Ki, μM |
|---|---|---|---|---|
| 14 | 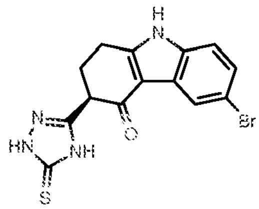 | 1010 | -6.90 | 8.82 |
| 15 | 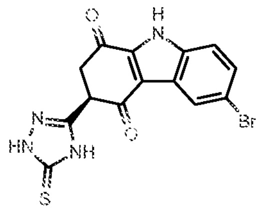 | 1013 | -6.86 | 9.34 |
| 16 | 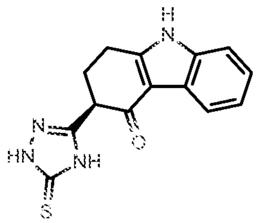 | 1008 | -6.83 | 9.81 |
| 17 | 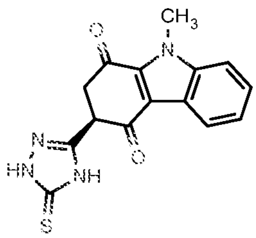 | 1018 | -6.79 | 10.62 |
| 18 | 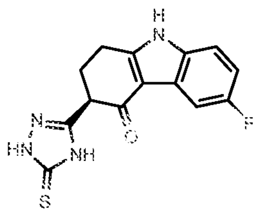 | 1009 | -6.74 | 11.52 |
| 19 | 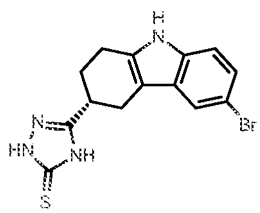 | 1007 | -6.57 | 15.27 |
| 20 | 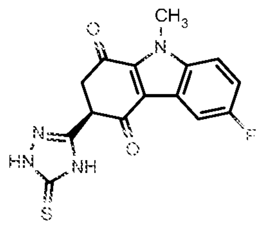 | 1019 | -6.50 | 17.27 |

(continued)

| | Compound | CdId | AD, kcal/mol | Ki, µM |
|---|---|---|---|---|
| 21 | 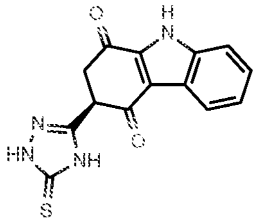 | 1011 | -6.44 | 18.87 |
| 22 | 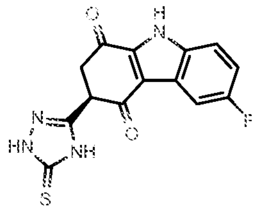 | 1012 | -6.34 | 22.47 |
| 23 | 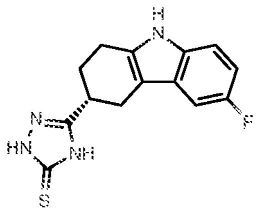 | 1006 | -6.32 | 23.18 |
| 24 | 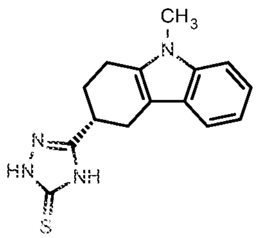 | 1014 | -6.26 | 25.68 |
| 25 | 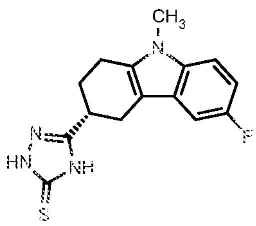 | 1015 | -6.09 | 34.29 |

**SOURCES:**

ADDIN EN.REFLIST Adler V., Yin Z., et al. (1999). "Regulation of JNK signaling by GSTp." The EMBO Journal 18(5): 1321-1334.

Armstrong R. N. (1997). "Structure, Catalytic Mechanism, and Evolution of the Glutathione Transferases." Chemical Research in Toxicology 10(1): 2-18.

Berman H. M., Westbrook J., et al. (2000). "The Protein Data Bank." Nucleic acids research 28(1): 235-242.

Burg D. and Mulder G. J. (2002). "Glutathione conjugates and their synthetic derivatives as inhibitors of glutathione-dependent enzymes involved in cancer and drug resistance." Drug Metabolism Reviews 34(4): 821-863.

Caccuri A. M., Ascenzi P., et al. (1996). "Structural Flexibility Modulates the Activity of Human Glutathione Transferase P1-1:Influence of a poor co-substrate on dynamics and kinetics of human glutathione transferase." Journal of Biological Chemistry 271(27): 16193-16198.

Cui H., Shen J., et al. (2008). "4-Aryl-1,3,2-oxathiazolylium-5-olate: a novel GST inhibitor to release JNK and activate c-Jun for cancer therapy." Cancer Chemotherapy and Pharmacology 62(3): 509-515.

Damia G. and D'Incalci M. (1998). "Mechanisms of resistance to alkylating agents." Cytotechnology 27(1-3): 165-173.

De Luca A., Federici L., et al. (2012). "New Insights into the Mechanism of JNK1 Inhibition by Glutathione Transferase P1-1." Biochemistry 51(37): 7304-7312.

Flatgaard J., Bauer K., et al. (1993). "Isozyme specificity of novel glutathione-S-transferase inhibitors." Cancer Chemotherapy and Pharmacology 33(1): 63-70.

Hayes J. D., Flanagan J. U., et al. (2005). "Glutathione transferases." Annual Review of Pharmacology and Toxicology 45(1): 51-88.

Jedlitschky G., Leier I., et al. (1994). "ATP-dependent Transport of Glutathione S-Conjugates by the Multidrug Resistance-associated Protein." Cancer Research 54(18): 4833-4836.

Mannervik B., Board P. G., et al. (2005). Nomenclature for Mammalian Soluble Glutathione Transferases. Methods in Enzymology. S. Helmut and P. Lester, Academic Press. Volume 401: 1-8.

Meijerman I., Beijnen J. H., et al. (2008). "Combined action and regulation of phase II enzymes and multidrug resistance proteins in multidrug resistance in cancer." Cancer Treatment Reviews 34(6): 505-520.

Morrow C. S., Smitherman P. K., et al. (1998). "Coordinated Action of Glutathione S-Transferases (GSTs) and Multidrug Resistance Protein 1 (MRPI) in Antineoplastic Drug Detoxification: MechaniSm of GST a1-1- and MRP1-associated resistance to chlorambucil in MCF7 breast carcinoma cells." Journal of Biological Chemistry 273(32): 20114-20120.

Mukanganyama S., Widersten M., et al. (2002). "Inhibition of glutathione S-transferases by antimalarial drugs possible implications for circumventing anticancer drug resistance." International Journal of Cancer 97(5): 700-705.

Mulder T., Verspaget, HW., Sier, CFM., Roelofs, HMJ., Ganesh, S., Griffioen, G., Peters, VHM. (1995). "Glutathione S-Transferase TTin Colorectal Tumors Is Predictive for Overall Survival." Cancer Research 55: 2696-2702.

Ricci G., De Maria F., et al. (2005). "7-Nitro-2,1,3-benzoxadiazole Derivatives, a New Class of Suicide Inhibitors for Glutathione S-Transferases:Mechanism of action of potential anticancer drugs." Journal of Biological Chemistry 280(28): 26397-26405.

Rotili D., De Luca A., et al. (2015). "Synthesis and structure-activity relationship of new cytotoxic agents targeting human glutathione-S-transferases." European Journal of Medicinal Chemistry 89: 156-171.

Sato K. (1989). "Glutathione transferases as markers of preneoplasia and neoplasia." Adv Cancer Res. 52: 205-255.

Schultz M., Dutta S., et al. (1997). "Inhibitors of glutathione S-transferases as therapeutic agents." Advanced Drug Delivery Reviews 26(2-3): 91-104.

Tew K. D. (1994). "Glutathione-associated Enzymes in Anticancer Drug Resistance." Cancer Research 54(16): 4313-4320.

Tew K. D. (2005). "TLK-286: a novel glutathione S-transferase-activated prodrug." Expert Opinion on Investigational Drugs 14(8): 1047-1054.

Tew K. D. (2007). "Redox in redux:Emergent roles for glutathione S-transferase P (GSTP) in regulation of cell signaling and S-glutathionylation." Biochemical Pharmacology 73(9): 1257-1269.

Tew K. D., Bomber A. M., et al. (1988). "Ethacrynic Acid and Piriprost as Enhancers of Cytotoxicity in Drug Resistant and Sensitive Cell Lines." Cancer Research 48(13): 3622-3625.

Tidefelt U., Elmhorn-Rosenborg A., et al. (1992). "Expression of glutathione transferase pi as a predictor for treatment results at different stages of acute nonlymphoblastic leukemia." Cancer Res 52(12): 3281-3285.

Townsend D. M. and Tew K. D. (2003). "The role of glutathione-S-transferase in anti-cancer drug resistance." Oncogene 22(47): 7369-7375.

Turella P., Cerella C., et al. (2005). "Proapoptotic Activity of New Glutathione S-Transferase Inhibitors." Cancer Research 65(9): 3751-3761.

Wang T., Arifoglu P., et al. (2001). "Glutathione S-transferase P1-1 (GSTP1-1) inhibits c-Jun N-terminal kinase (JNK1) signaling through interaction with the C terminus." J Biol Chem 276(24): 20999-21003.

Zheng J., Liu, G., Tozkoparan, B., Wen, D. (2005). "Mechanistic Studies of Inactivation of Glutathione S-transferase Pi Isozyme by a Haloenol Lactone Derivative." Medicinal Chemistry 1(2): 191-198.

**OTHER ARTICLES AND PATENTS RELATED TO THE SUBJECT MATTER:**

**ARTICLES**

1. Tu GG, Yan YG, Chen XY, Lv QL, Wang JQ, Li SH, Synthesis and antiproliferative assay of 1,3,4-oxadiazole and 1,2,4-triazole derivatives in cancer cells, Drug Discoveries & Therapeutics. 2013; 7(2):58-65.

2. Gadegoni H, Manda S, Synthesis and screening of some novel substituted indoles contained 1,3,4-oxadiazole and 1,2,4-triazole moiety, Chinese Chemical Letters 24 (2013) 127-130.

3. Plech T, Wujec M, Kosikowska U, Malm A, Rajtar B, Polz-Dacewicz M., Synthesis and in vitro activity of 1,2,4-triazole-ciprofloxacin hybrids against drug-susceptible and drug-resistant bacteria, European Journal of Medicinal Chemistry 60 (2013) 128-134.

4. Wittine K, Stipković Babić M, Makuc D, Plavec J, Kraljević Pavelić S, Sedić M, Pavelić K, Leyssen P, Neyts J, Balzarini J, Mintas M., Novel 1,2,4-triazole and imidazole derivatives of L-ascorbic and imino-ascorbic acid: synthesis, anti-HCV and antitumor activity evaluations, Bioorganic & Medicinal Chemistry 20 (2012) 3675-3685.

5. Plech T, Luszczki JJ, Wujec M, Flieger J, Pizoń M, Synthesis, characterization and preliminary anticonvulsant evaluation of some 4-alkyl-1,2,4-triazoles, European Journal of Medicinal Chemistry 60 (2013) 208-215.

6. Plech T, Wujec M, Siwek A, Kosikowska U, Malm A., Synthesis and antimicrobial activity of thiosemicarbazides, s-triazoles and their Mannich bases bearing 3-chlorophenyl moiety, European Journal of Medicinal Chemistry 46 (2011) 241-248.

7. Shivarama Holla B, Veerendra B, Shivananda MK, Poojary B., Synthesis characterization and anticanceractivitystudies on some Mannichbasesderived from 1,2,4-triazoles, European Journal of Medicinal Chemistry 38 (2003) 759-767.

8. Moulin A, Brunel L, Boeglin D, Demange L, Ryan J, M'Kadmi C, Denoyelle S, Martinez J, Fehrentz JA., The 1,2,4-triazole as a scaffold for the design of ghrelin receptor ligands: development of JMV 2959, a potent antagonist, Amino Acids (2013) 44:301-314.

**PATENTS:**

9. WO2014/048065, Triazolyl derivatives as SYK inhibitors.

10. WO2006/095783A1, Novel HSP90 Inhibitor.

11. WO2011/128381 A1, Triazole Compounds As KSP Inhibitors.

12. WO2012/035124A1, Novel triazole derivatives with improved receptor activity and bioavailability properties as ghrelin antagonists of growth hormone secretagogue receptors.

13. WO2014/041106, Triazole carboxamide derivatives.

14. WO2006/055760A9, Triazole compounds that modulate hsp90 activity.

15. WO2014/012614_Fused triazole derivatives as gamma secretase modulators.

16. WO2004/093874, Use of 7-Nitro-2,1,3 benzoxadiazole derivatives for anticancer therapy.

17. US 2012/0053136 A1, Diagnosis and treatment of drug-resistant Ewing's sarcoma.

18. WO 2006097472 A2, Novel inhibitors of glutathione-s-transferase

19. US 6495370 B1 ,Inhibition of glutathione transferase by haloenol lactones

## Claims

**1.** A compound ,with one of the general formulas

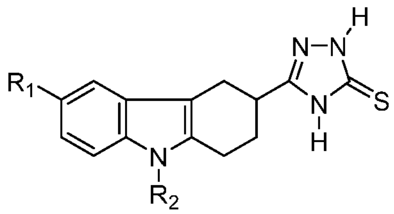
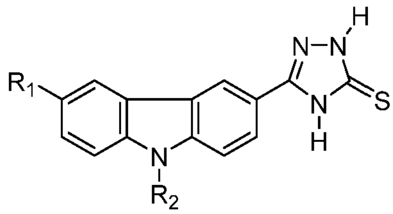

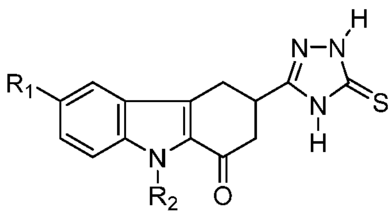
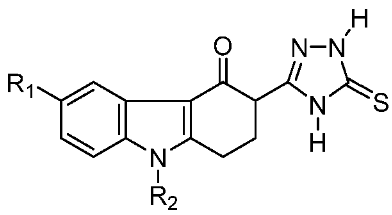

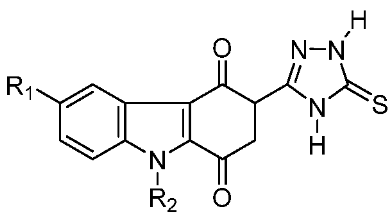

wherein,

$R_1$ is -H, -Br or -F,
$R_2$ is -H or $-CH_3$

**2.** A compound according to claim 1, **characterized by** being selected from the below mentioned group.

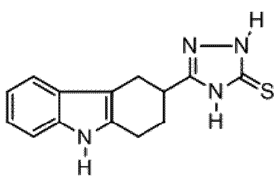
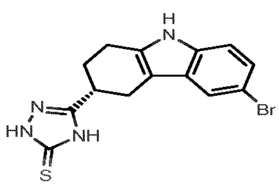
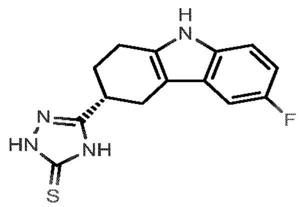

**3.** A compound according to claim 1, **characterized by** being selected from the below mentioned group.

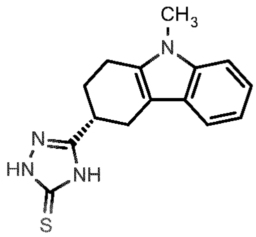

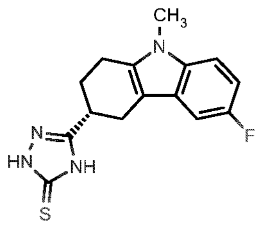

4. A compound according to claim 1, **characterized by** being selected from the below mentioned group.

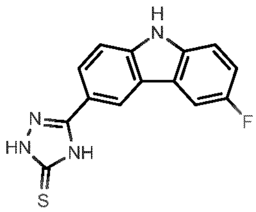

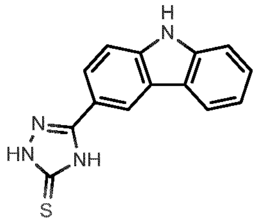

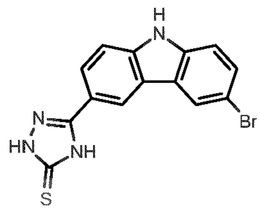

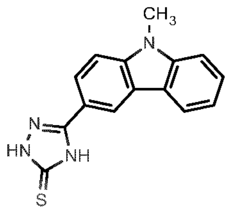

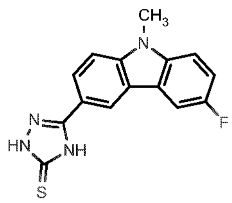

5. A compound according to claim 1, **characterized by** being selected from the below mentioned group.

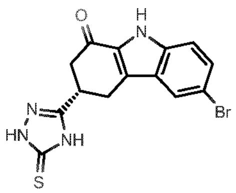

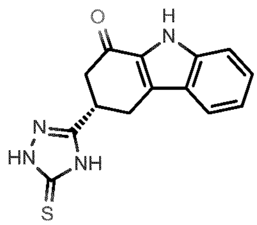

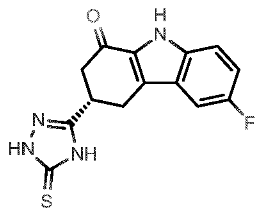

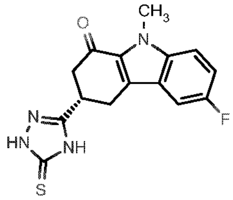

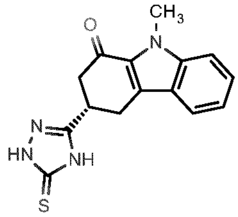

6. A compound according to claim 1, **characterized by** being selected from the below mentioned group.

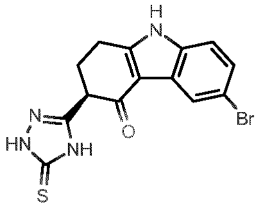

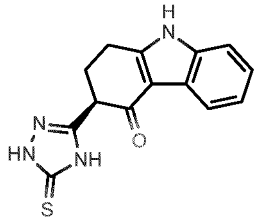

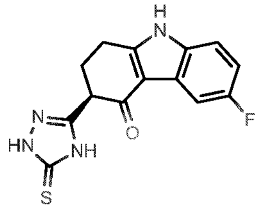

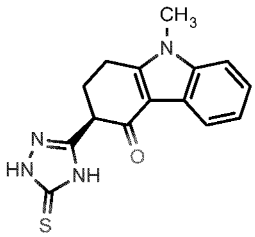
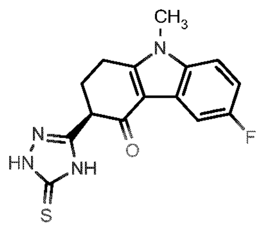

7. A compound according to claim 1, **characterized by** being selected from the below mentioned group.

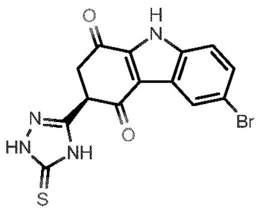
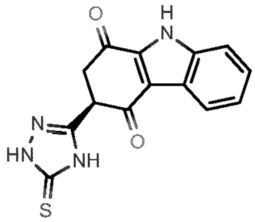
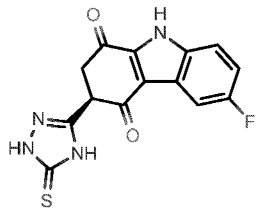

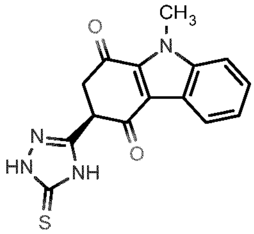
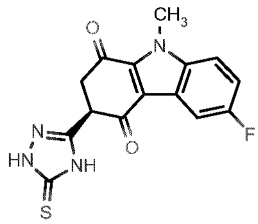

8. A method for synthesizing a compound according to claim 2, **characterized by** comprising the below mentioned process step

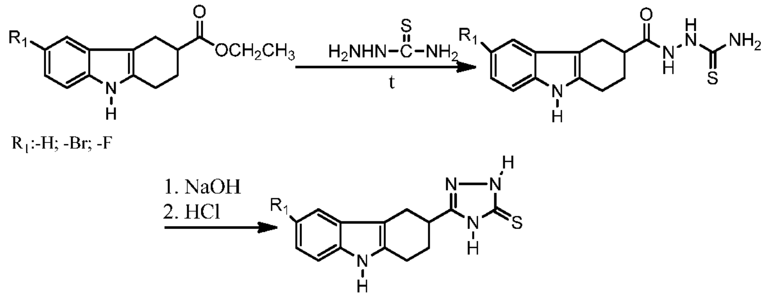


9. A method for synthesizing a compound according to claim 3, **characterized by** comprising the below mentioned process step.

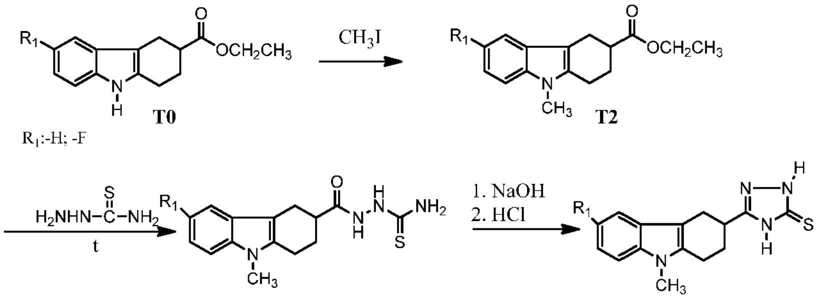


10. A method for synthesizing a compound according to claim 4, **characterized by** comprising the below mentioned

process steps

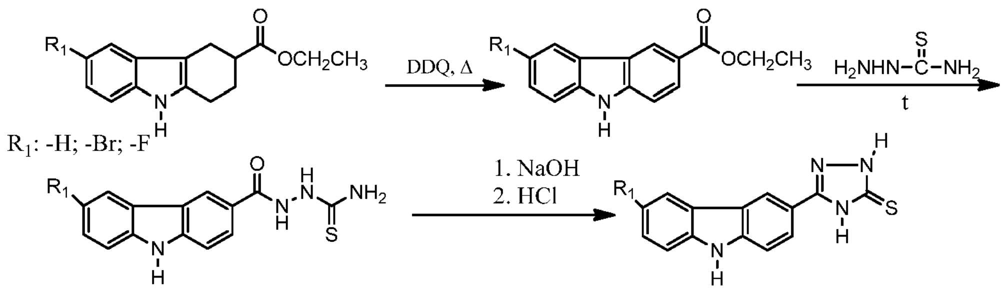


Or

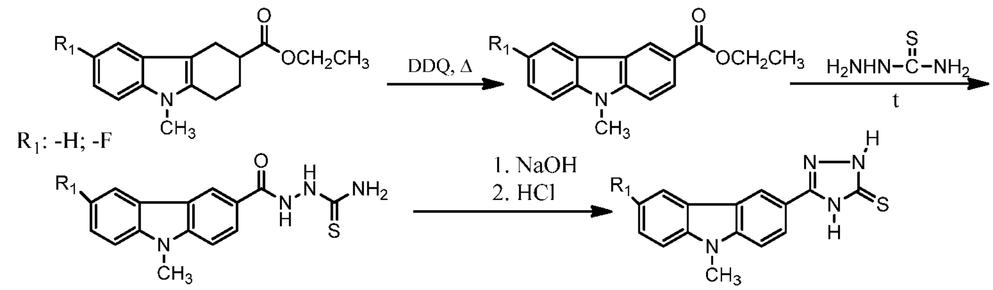


**11.** A method for synthesizing a compound according to claim 5, **characterized by** comprising the below mentioned process step

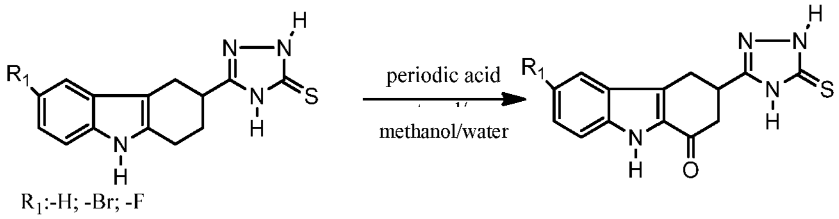


Or

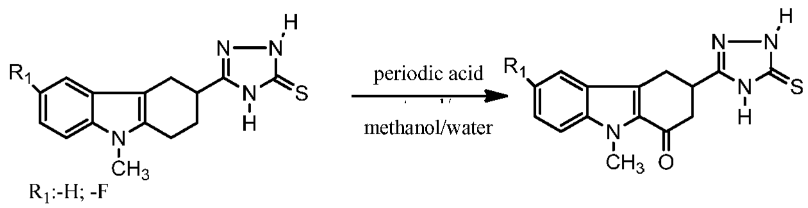


**12.** A method for synthesizing a compound according to claim 6, **characterized by** comprising the below mentioned process step respectively.

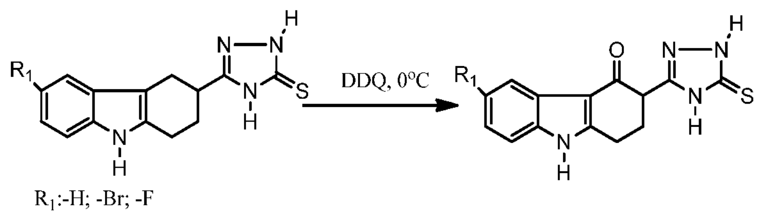

**or**

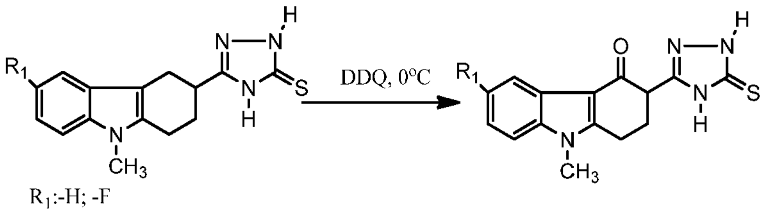


13. A method for synthesizing a compound according to claim 7, **characterized by** comprising the below mentioned process step respectiveley.

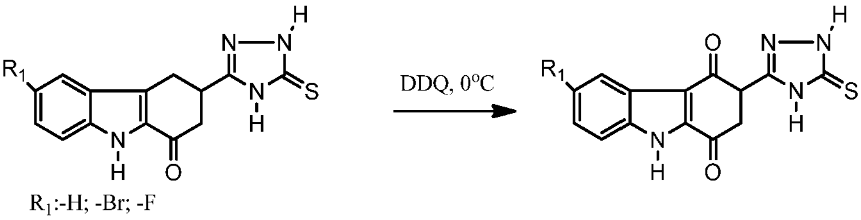


**or**

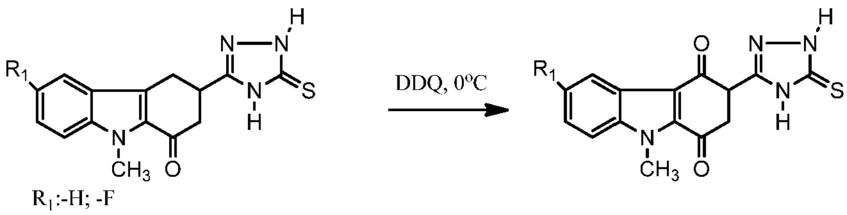


14. A compound according to any of claims 1-7 for use in treatment.

15. A pharmaceutical composition comprising a compound according to any of claims 1-7.

**Patentansprüche**

1. Verbindung mit einer der folgenden allgemeinen Formeln

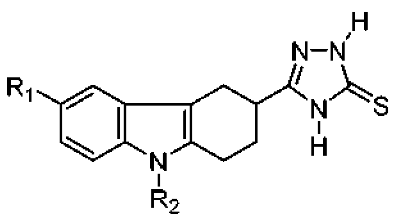

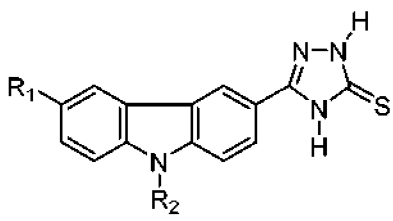

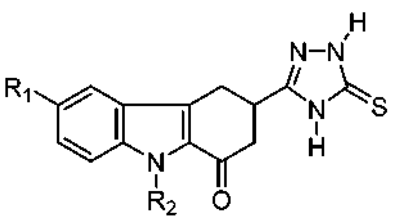

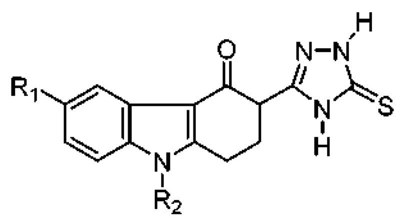

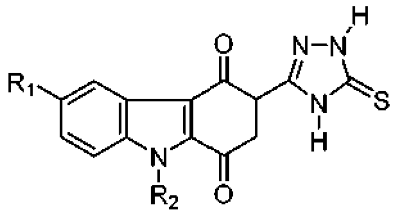

wobei,

$R_1$ ist -H, -Br oder -F,
$R_2$ ist -H oder $-CH_3$

2. Verbindung nach Anspruch 1, **dadurch gekennzeichnet, dass** sie aus der unten genannten Gruppe ausgewählt ist.

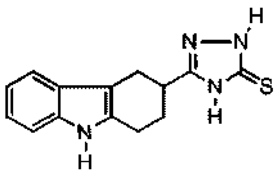
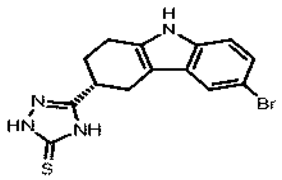
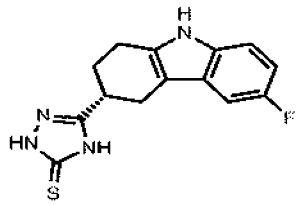

3. Verbindung nach Anspruch 1, **dadurch gekennzeichnet, dass** sie aus der unten genannten Gruppe ausgewählt ist.

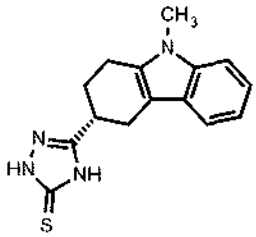
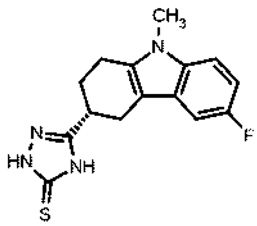

4. Verbindung nach Anspruch 1, **dadurch gekennzeichnet, dass** sie aus der unten genannten Gruppe ausgewählt ist.

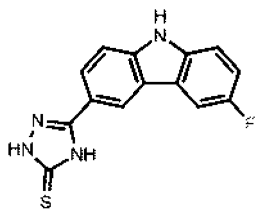
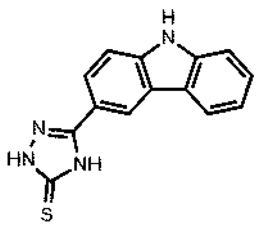
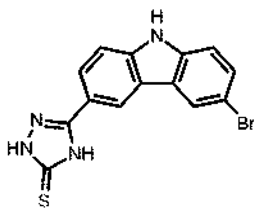
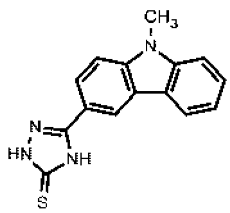
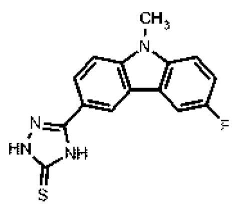

5. Verbindung nach Anspruch 1, **dadurch gekennzeichnet, dass** sie aus der unten genannten Gruppe ausgewählt ist.

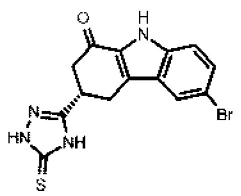 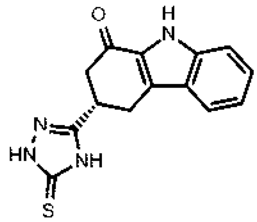 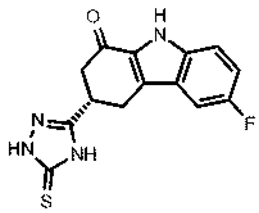

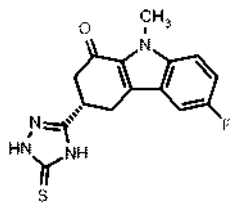 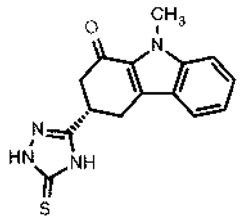

6. Verbindung nach Anspruch 1, **dadurch gekennzeichnet, dass** sie aus der unten genannten Gruppe ausgewählt ist.

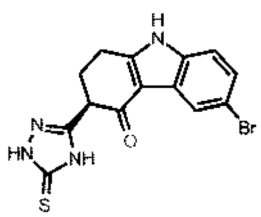 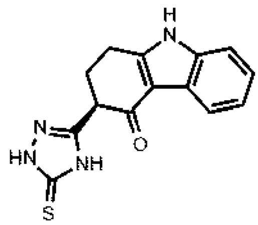 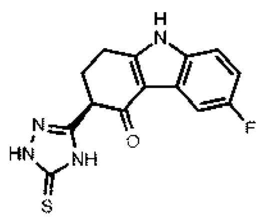

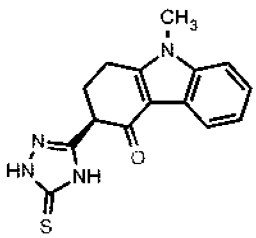 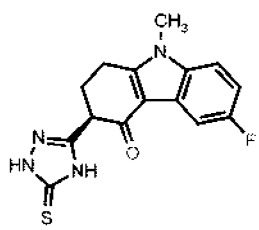

7. Verbindung nach Anspruch 1, **dadurch gekennzeichnet, dass** sie aus der unten genannten Gruppe ausgewählt ist.

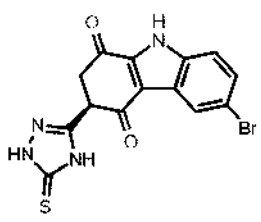 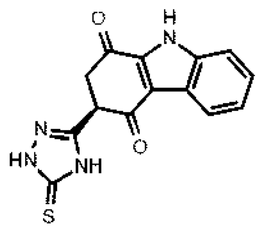 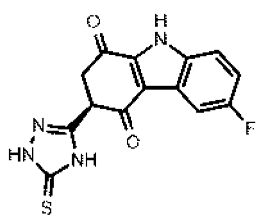

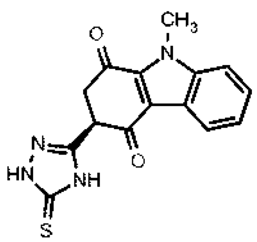 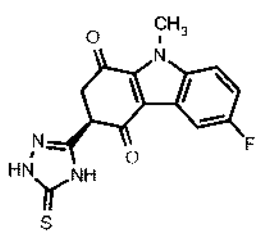

8. Verfahren zur Synthese einer Verbindung nach Anspruch 2, **dadurch gekennzeichnet, dass** es den unten ge-

nannten Prozessschritt umfasst

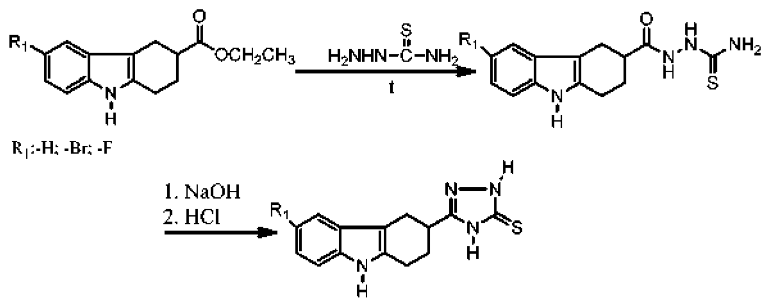


9. Verfahren zur Synthese einer Verbindung nach Anspruch 3, **dadurch gekennzeichnet, dass** es den unten genannten Prozessschritt umfasst.

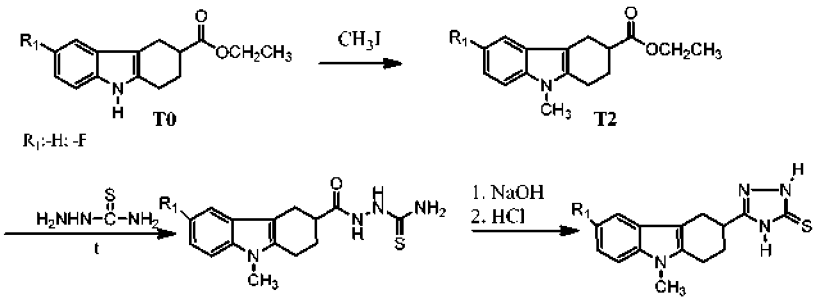


10. Verfahren zur Synthese einer Verbindung nach Anspruch 4, **dadurch gekennzeichnet, dass** es den unten genannten Prozessschritt umfasst

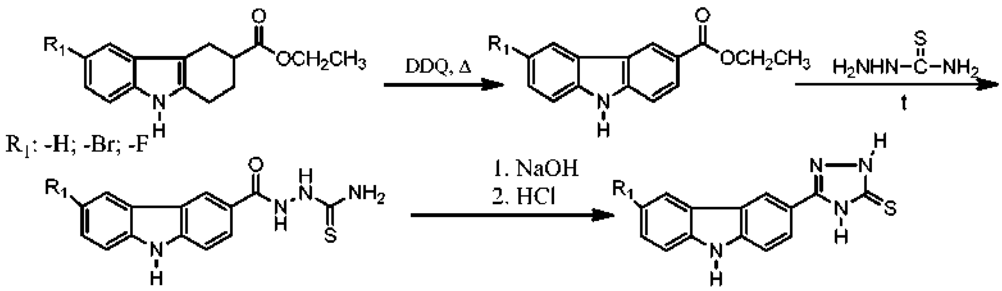


oder

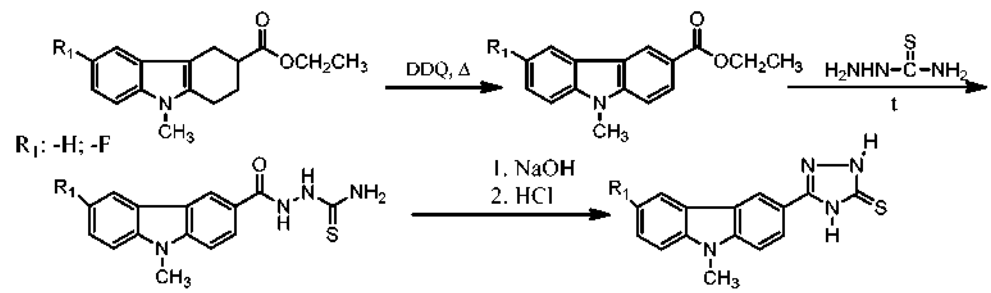


11. Verfahren zur Synthese einer Verbindung nach Anspruch 5, **dadurch gekennzeichnet, dass** es den unten genannten Prozessschritt umfasst

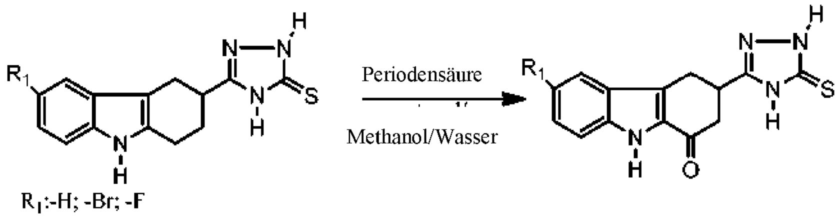


oder

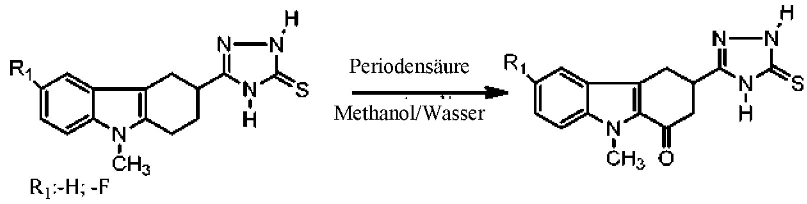


**12.** Verfahren zur Synthese einer Verbindung nach Anspruch 6, **dadurch gekennzeichnet, dass** es den jeweils unten genannten Prozessschritt umfasst.

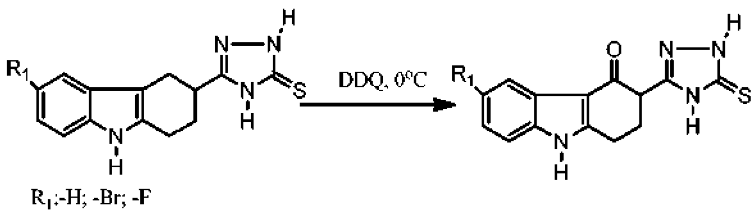


oder

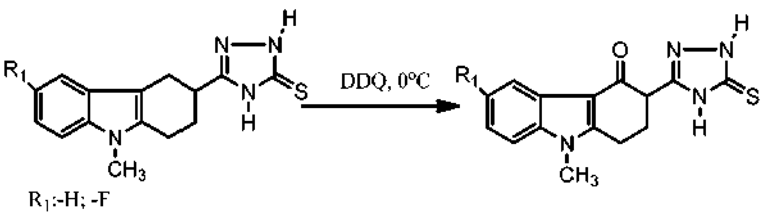


**13.** Verfahren zur Synthese einer Verbindung nach Anspruch 7, **dadurch gekennzeichnet, dass** es den jeweils unten genannten Prozessschritt umfasst.

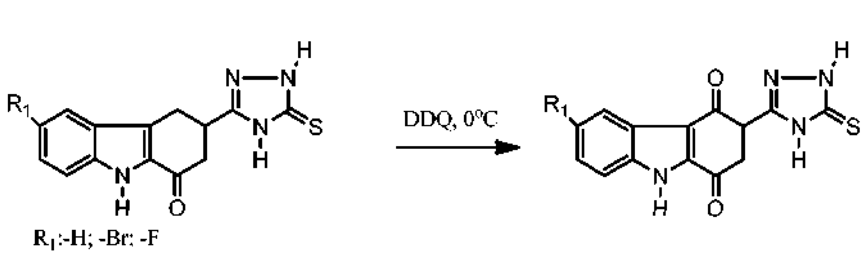


oder

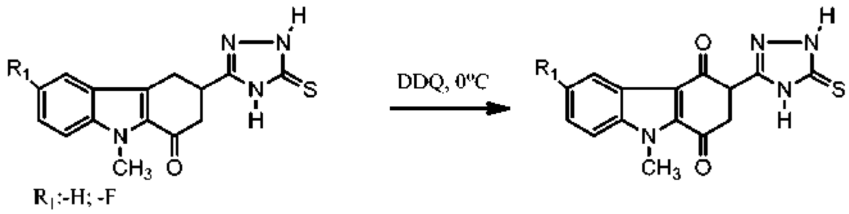


**14.** Verbindung nach einem der Ansprüche 1-7 zur Verwendung bei der Behandlung.

**15.** Pharmazeutische Zusammensetzung, umfassend eine Verbindung nach einem der Ansprüche 1-7.

**Revendications**

**1.** Composé ayant l'une des formules générales suivantes

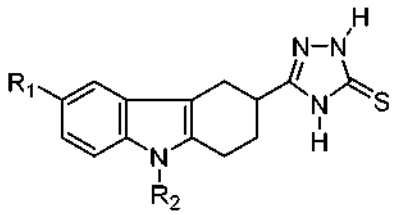

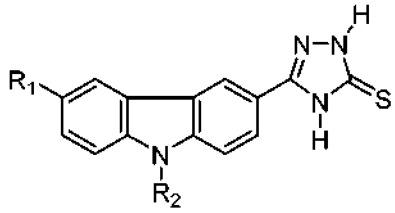

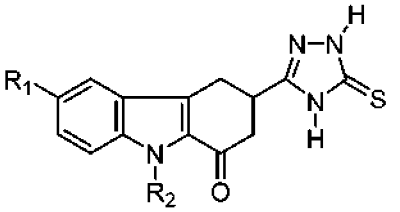

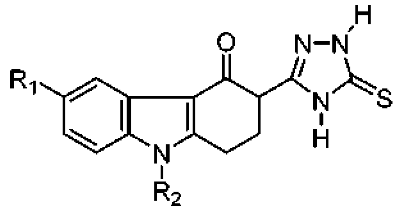

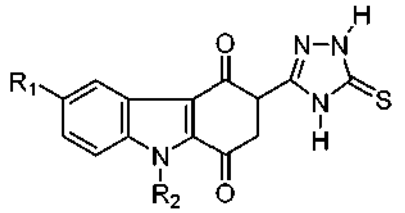

dans lequel,

$R_1$ est -H, -Br ou -F,
$R_2$ est -H ou $-CH_3$

**2.** Composé selon la revendication 1, **caractérisé en ce qu'**il est choisi dans le groupe mentionné ci-dessous.

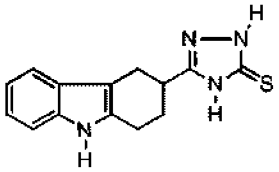

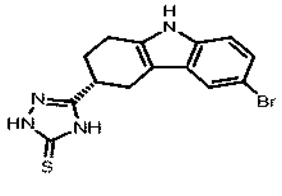

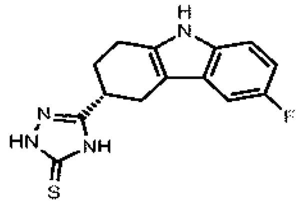

**3.** Composé selon la revendication 1, **caractérisé en ce qu'**il est choisi dans le groupe mentionné ci-dessous.

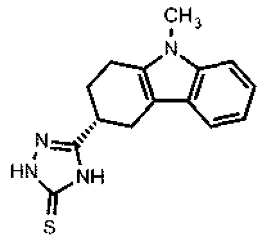

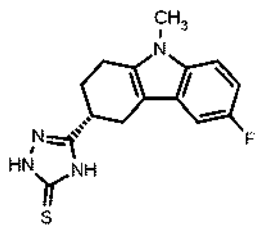

4. Composé selon la revendication 1, **caractérisé en ce qu'**il est choisi dans le groupe mentionné ci-dessous.

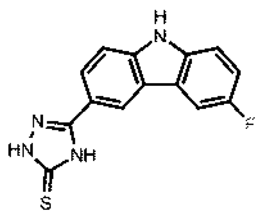

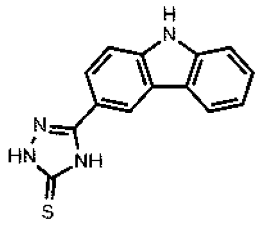

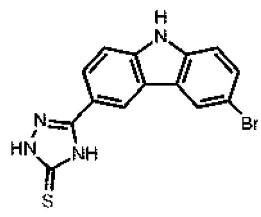

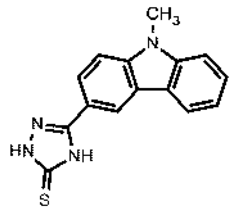

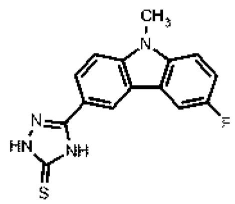

5. Composé selon la revendication 1, **caractérisé en ce qu'**il est choisi dans le groupe mentionné ci-dessous.

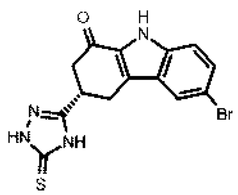

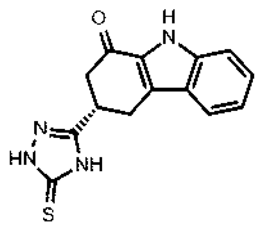

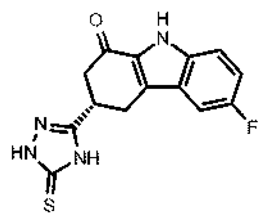

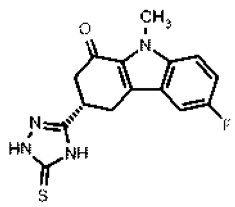

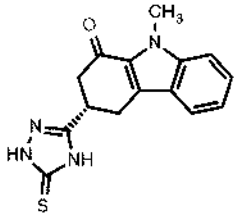

6. Composé selon la revendication 1, **caractérisé en ce qu'**il est choisi dans le groupe mentionné ci-dessous.

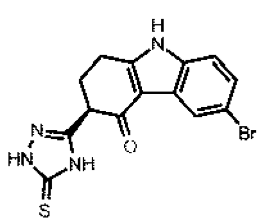

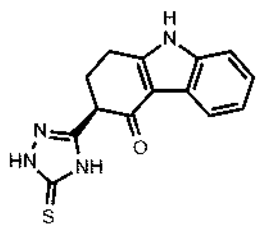

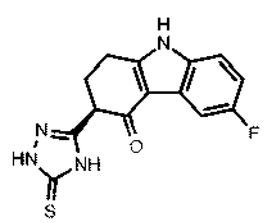

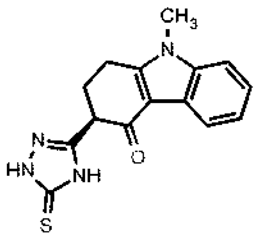

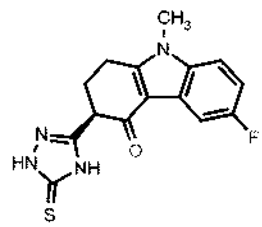

**7.** Composé selon la revendication 1, **caractérisé en ce qu'**il est choisi dans le groupe mentionné ci-dessous.

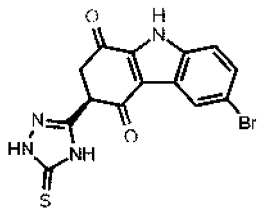

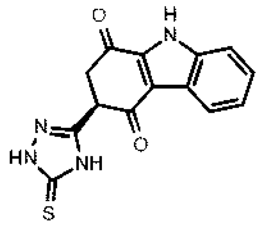

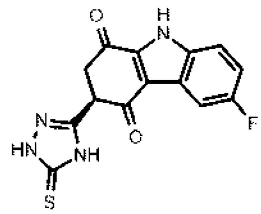

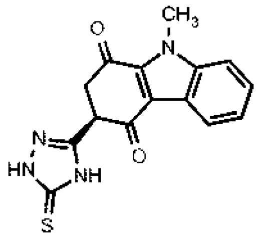

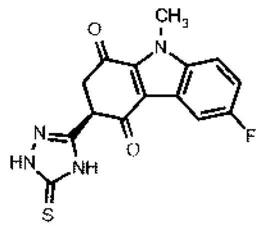

**8.** Méthode de synthèse d'un composé selon la revendication 2, **caractérisée en ce qu'**elle comprend l'étape du processus mentionnée ci-dessous

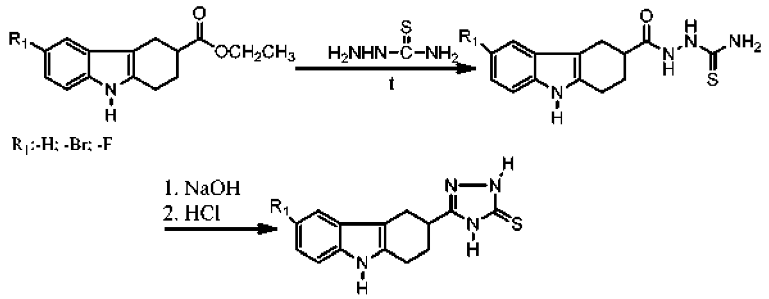


**9.** Méthode de synthèse d'un composé selon la revendication 3, **caractérisée en ce qu'**elle comprend l'étape du processus mentionnée ci-dessous.

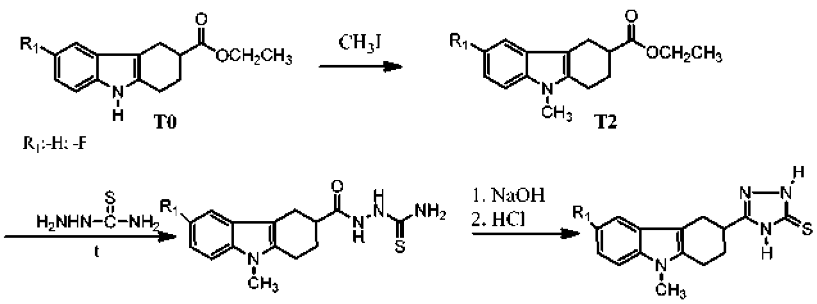


**10.** Méthode de synthèse d'un composé selon la revendication 4, **caractérisée en ce qu'**elle comprend les étapes du

processus mentionnées ci-dessous

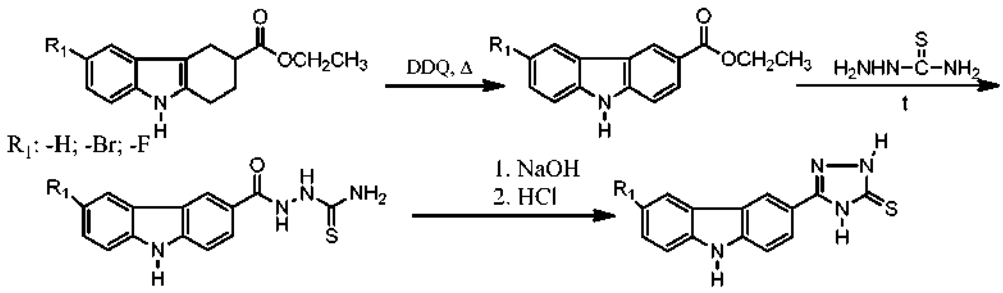


Ou

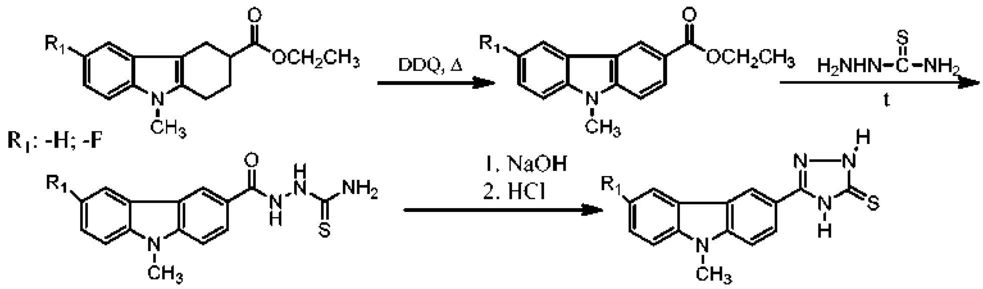


**11.** Méthode de synthèse d'un composé selon la revendication 5, **caractérisée en ce qu'**elle comprend l'étape du processus mentionnée ci-dessous

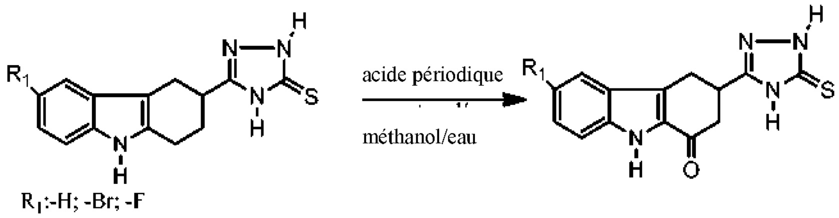


Ou

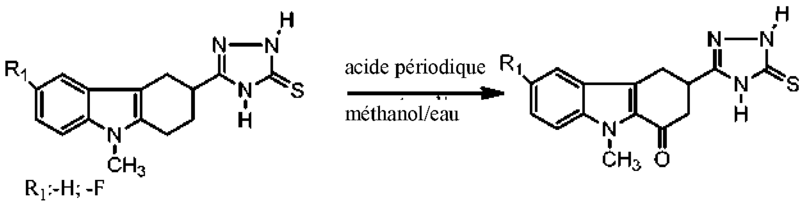


**12.** Méthode de synthèse d'un composé selon la revendication 6, **caractérisée en ce qu'**elle comprend l'étape du processus mentionnée ci-dessous respectivement.

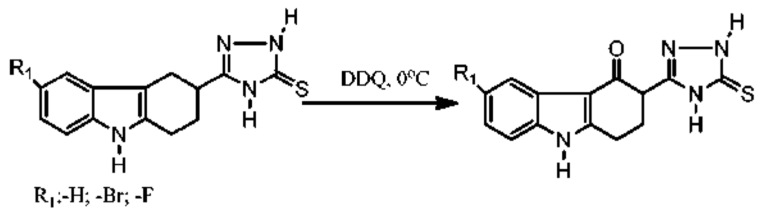

Ou

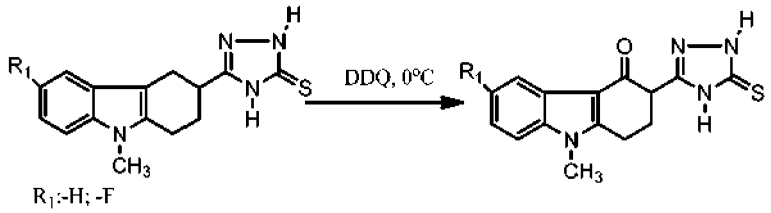


13. Méthode de synthèse d'un composé selon la revendication 7, **caractérisée en ce qu'**elle comprend l'étape du processus mentionnée ci-dessous respectivement.

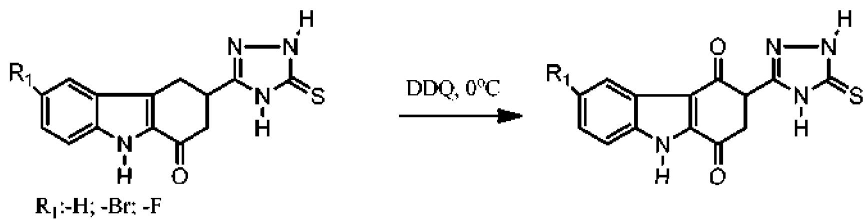


Ou

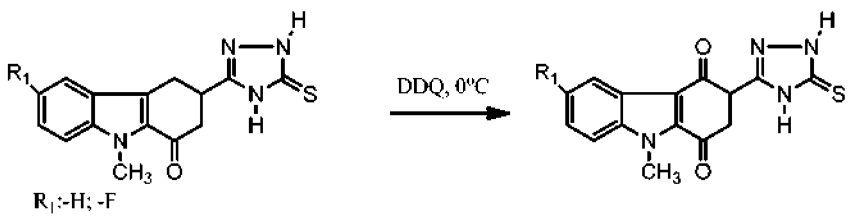


14. Composé selon l'une quelconque des revendications 1-7 pour utilisation dans le traitement.

15. Composition pharmaceutique comprenant un composé selon l'une quelconque des revendications 1-7.

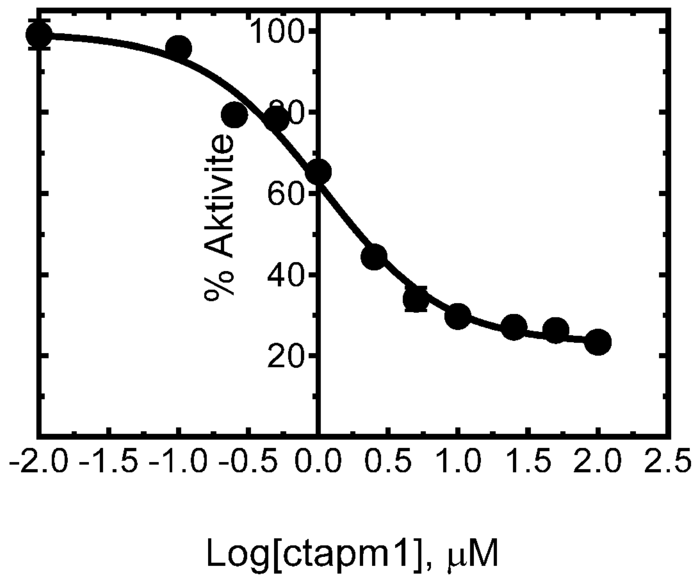
100
80
60
40
20
% Aktivite
-2.0 -1.5 -1.0 -0.5 0.0 0.5 1.0 1.5 2.0 2.5
Log[ctapm1], μM

**Figure 1.**

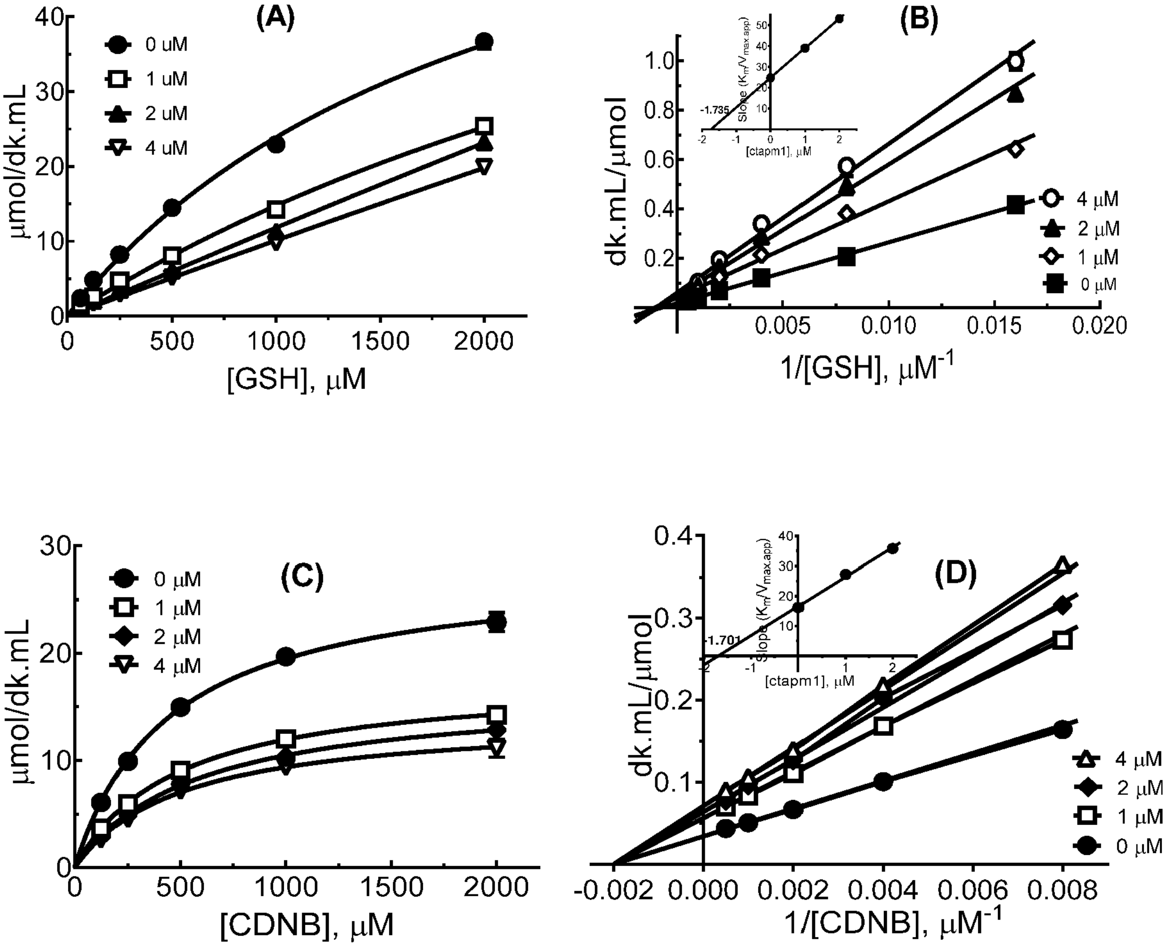
(A)
0 uM
1 uM
2 uM
4 uM
µmol/dk.mL
[GSH], µM
(B)
dk.mL/µmol
1/[GSH], µM-1
4 µM
2 µM
1 µM
0 µM
-1.735
Slope (Km/Vmax,app)
[ctapm1], µM
(C)
0 µM
1 µM
2 µM
4 µM
µmol/dk.mL
[CDNB], µM
(D)
dk.mL/µmol
1/[CDNB], µM-1
-1.701
[ctapm1], µM
4 µM
2 µM
1 µM
0 µM

Figure 2.

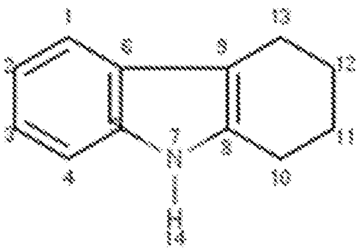

a

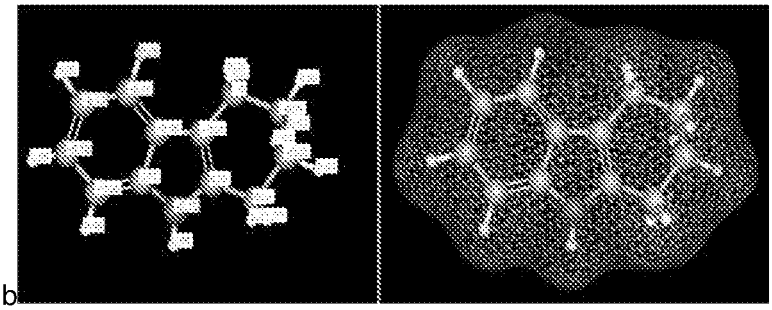

b

**Figure 3.**

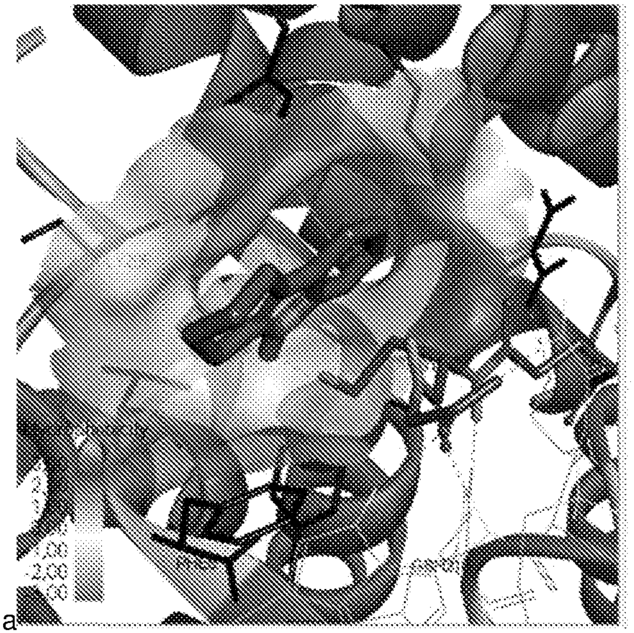

a

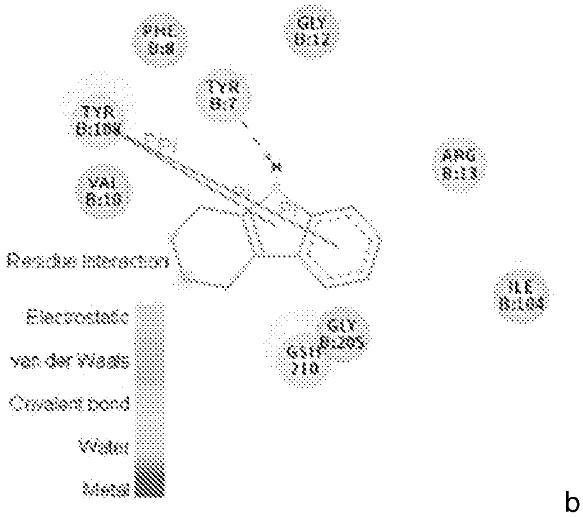


b

**Figure 4.**

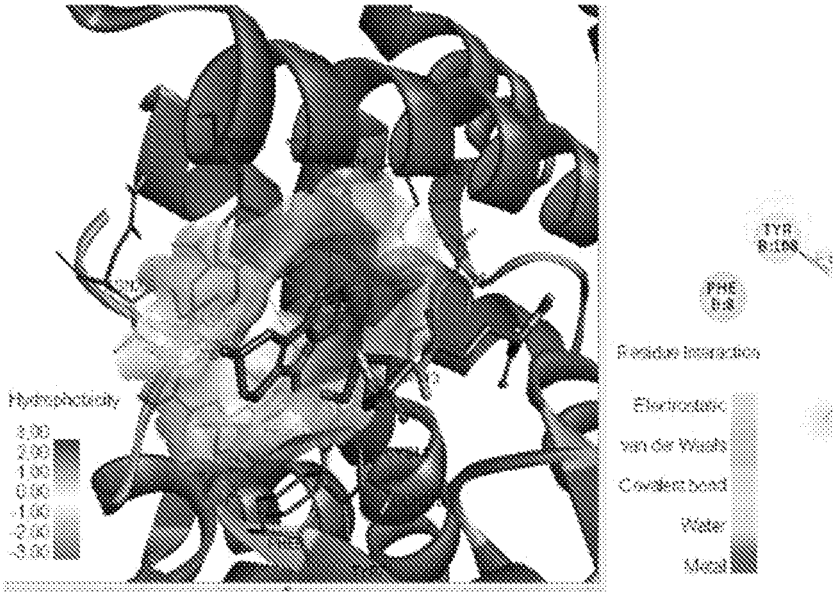


**Figure 5.**

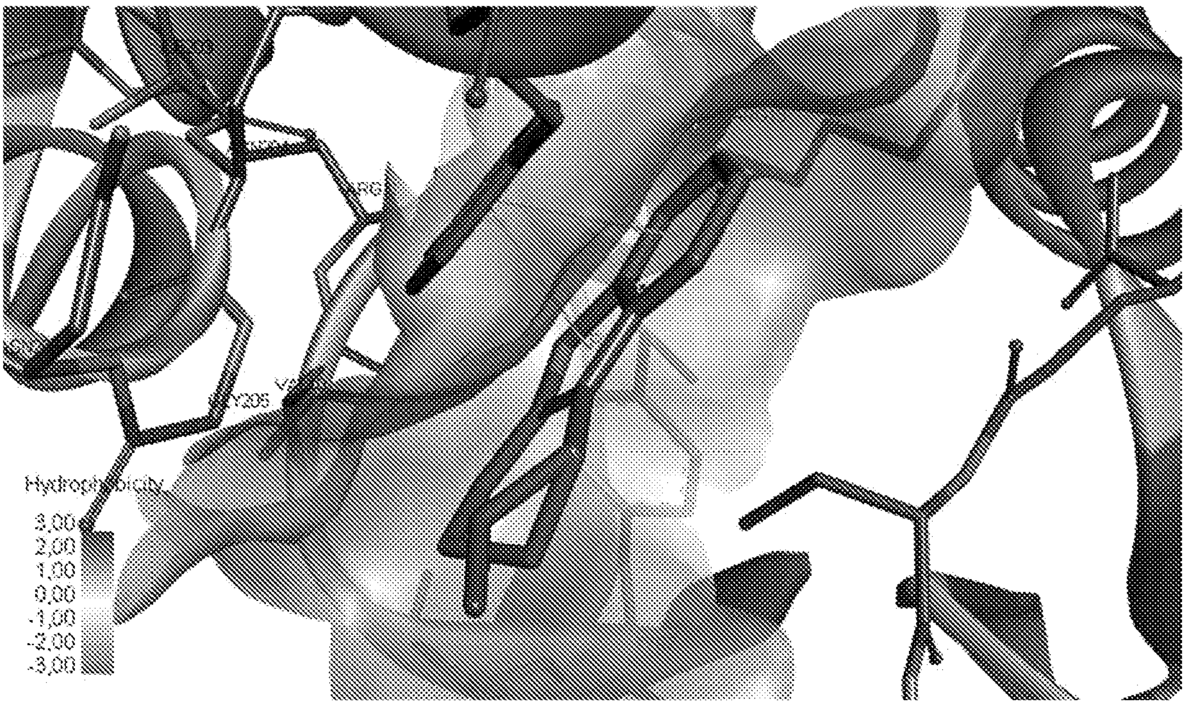


**Figure 6.**

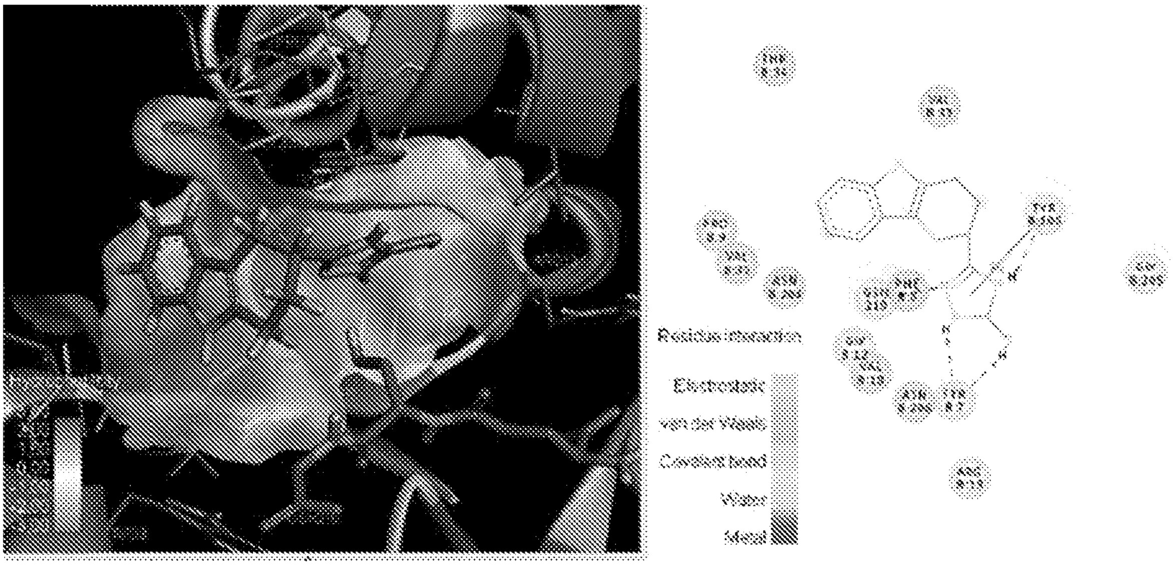


Figure 7.

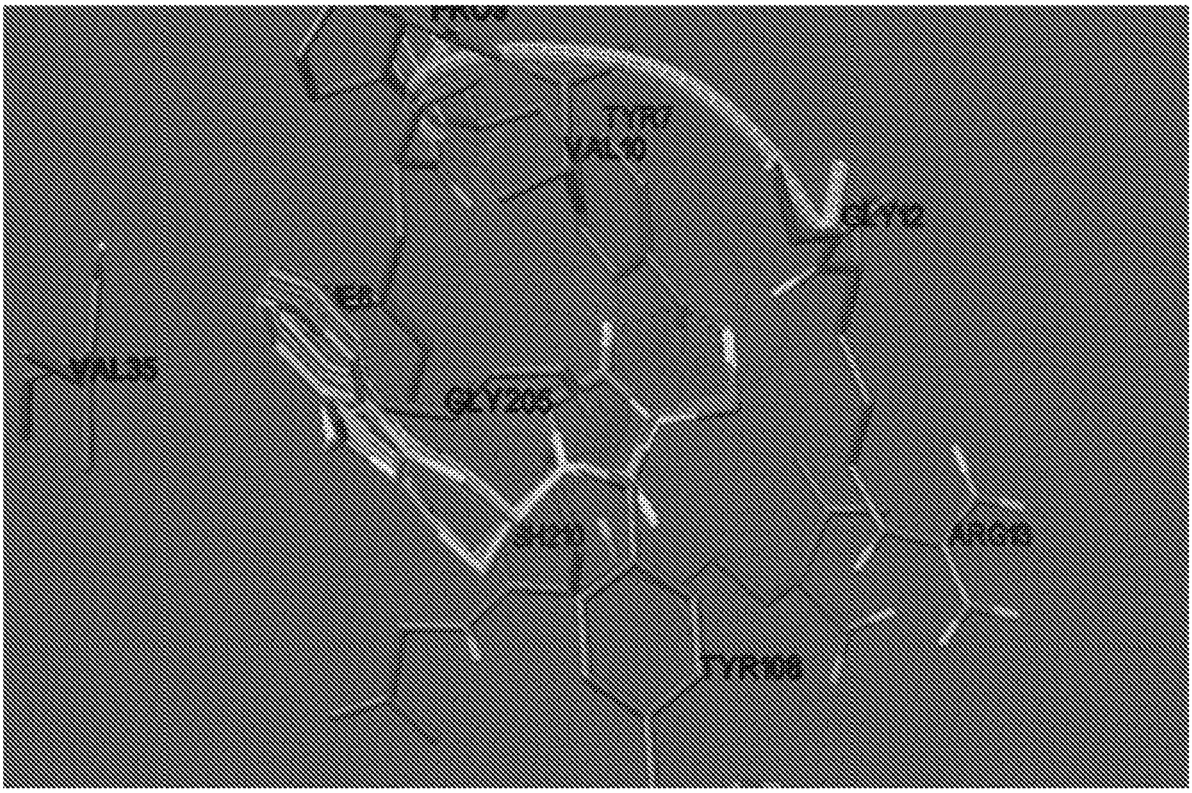


Figure 8.

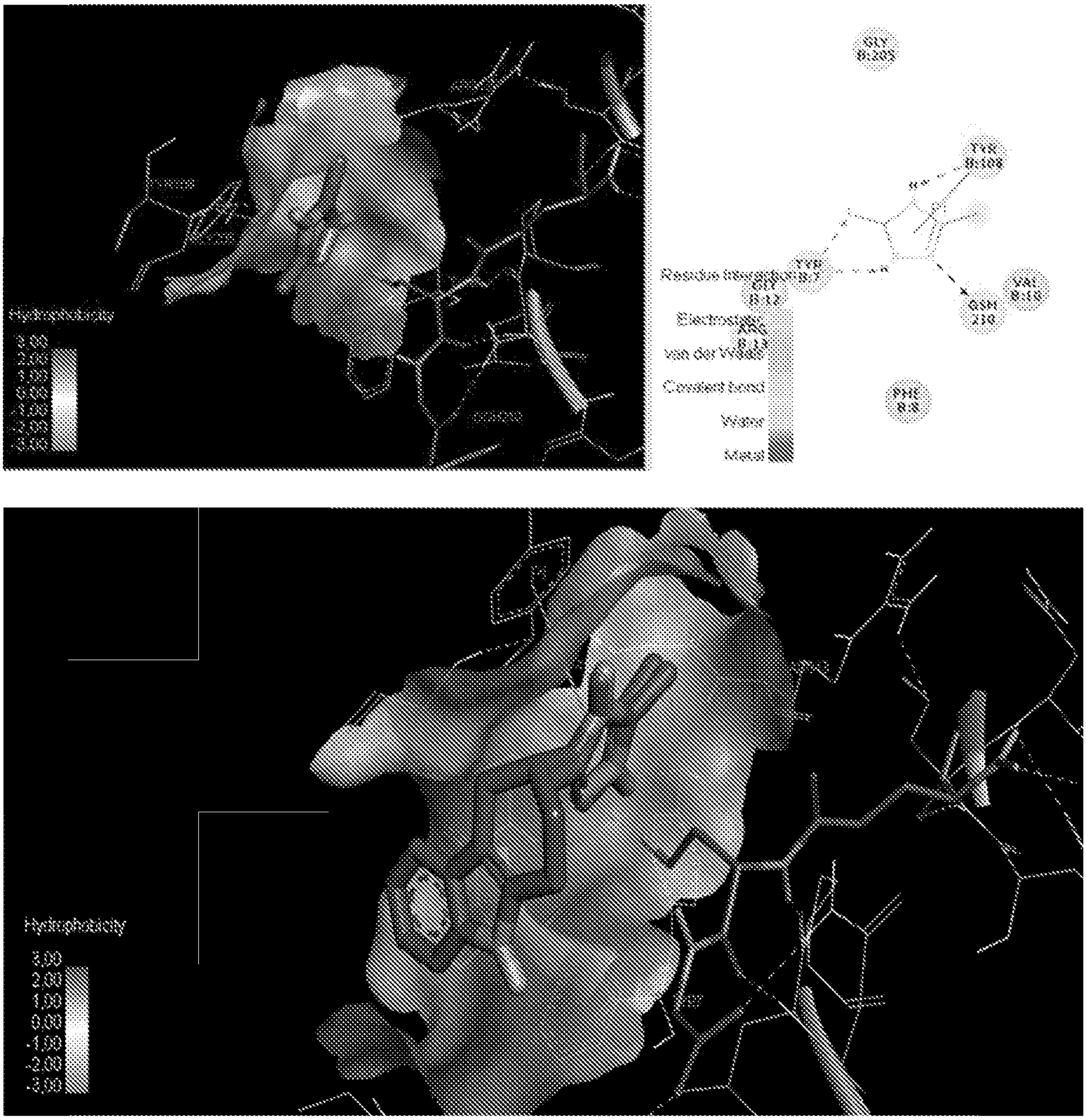
Hydrophobicity
3.00
2.00
1.00
0.00
-1.00
-2.00
-3.00
GLY B:205
TYR B:108
TYR B:7
GLY B:12
ARG B:13
VAL B:10
GSH 210
PHE B:8
Residue interaction
Electrostatic
van der Waals
Covalent bond
Water
Metal
Hydrophobicity
3.00
2.00
1.00
0.00
-1.00
-2.00
-3.00

A

B

Figure 9.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014048065 A
- WO 2006095783 A1
- WO 2011128381 A1
- WO 2012035124 A1
- WO 2014041106 A
- WO 2006055760 A9
- WO 2014012614 A
- WO 2004093874 A
- US 20120053136 A1
- WO 2006097472 A2
- US 6495370 B1


### Non-patent literature cited in the description


- **ADLER V. ; YIN Z. et al.** Regulation of JNK signaling by GSTp. *The EMBO Journal,* 1999, vol. 18 (5), 1321-1334
- **ARMSTRONG R. N.** Structure, Catalytic Mechanism, and Evolution of the Glutathione Transferases. *Chemical Research in Toxicology,* 1997, vol. 10 (1), 2-18
- **BERMAN H. M. ; WESTBROOK J. et al.** The Protein Data Bank. *Nucleic acids research,* 2000, vol. 28 (1), 235-242
- **BURG D. ; MULDER G. J.** Glutathione conjugates and their synthetic derivatives as inhibitors of glutathione-dependent enzymes involved in cancer and drug resistance. *Drug Metabolism Reviews,* 2002, vol. 34 (4), 821-863
- **CACCURI A. M. ; ASCENZI P. et al.** Structural Flexibility Modulates the Activity of Human Glutathione Transferase P1-1:Influence of a poor co-substrate on dynamics and kinetics of human glutathione transferase. *Journal of Biological Chemistry,* 1996, vol. 271 (27), 16193-16198
- **CUI H. ; SHEN J. et al.** 4-Aryl-1,3,2-oxathiazolylium-5-olate: a novel GST inhibitor to release JNK and activate c-Jun for cancer therapy. *Cancer Chemotherapy and Pharmacology,* 2008, vol. 62 (3), 509-515
- **DAMIA G. ; D'INCALCI M.** Mechanisms of resistance to alkylating agents. *Cytotechnology,* 1998, vol. 27 (1-3), 165-173
- **DE LUCA A. ; FEDERICI L. et al.** New Insights into the Mechanism of JNK1 Inhibition by Glutathione Transferase P1-1. *Biochemistry,* 2012, vol. 51 (37), 7304-7312
- **FLATGAARD J. ; BAUER K. et al.** Isozyme specificity of novel glutathione-S-transferase inhibitors. *Cancer Chemotherapy and Pharmacology,* 1993, vol. 33 (1), 63-70
- **HAYES J. D. ; FLANAGAN J. U. et al.** Glutathione transferases. *Annual Review of Pharmacology and Toxicology,* 2005, vol. 45 (1), 51-88
- **JEDLITSCHKY G. ; LEIER I. et al.** ATP-dependent Transport of Glutathione S-Conjugates by the Multidrug Resistance-associated Protein. *Cancer Research,* 1994, vol. 54 (18), 4833-4836
- Nomenclature for Mammalian Soluble Glutathione Transferases. **MANNERVIK B. ; BOARD P. G. et al.** Methods in Enzymology. Academic Press, 2005, vol. 401, 1-8
- **MEIJERMAN I. ; BEIJNEN J. H. et al.** Combined action and regulation of phase II enzymes and multidrug resistance proteins in multidrug resistance in cancer. *Cancer Treatment Reviews,* 2008, vol. 34 (6), 505-520
- **MORROW C. S. ; SMITHERMAN P. K. et al.** Coordinated Action of Glutathione S-Transferases (GSTs) and Multidrug Resistance Protein 1 (MRPI) in Antineoplastic Drug Detoxification: MechaniSm of GST a1-1- and MRP1-associated resistance to chlorambucil in MCF7 breast carcinoma cells. *Journal of Biological Chemistry,* 1998, vol. 273 (32), 20114-20120
- **MUKANGANYAMA S. ; WIDERSTEN M. et al.** Inhibition of glutathione S-transferases by antimalarial drugs possible implications for circumventing anticancer drug resistance. *International Journal of Cancer,* 2002, vol. 97 (5), 700-705
- **MULDER T. ; VERSPAGET, HW. ; SIER, CFM. ; ROELOFS, HMJ. ; GANESH, S. ; GRIFFIOEN, G. ; PETERS, VHM.** Glutathione S-Transferase TTin Colorectal Tumors Is Predictive for Overall Survival. *Cancer Research,* 1995, vol. 55, 2696-2702

- **RICCI G. ; DE MARIA F. et al.** 7-Nitro-2,1,3-benzoxadiazole Derivatives, a New Class of Suicide Inhibitors for Glutathione S-Transferases:Mechanism of action of potential anticancer drugs. *Journal of Biological Chemistry,* 2005, vol. 280 (28), 26397-26405
- **ROTILI D. ; DE LUCA A. et al.** Synthesis and structure-activity relationship of new cytotoxic agents targeting human glutathione-S-transferases. *European Journal of Medicinal Chemistry,* 2015, vol. 89, 156-171
- **SATO K.** Glutathione transferases as markers of preneoplasia and neoplasia. *Adv Cancer Res.,* 1989, vol. 52, 205-255
- **SCHULTZ M. ; DUTTA S. et al.** Inhibitors of glutathione S-transferases as therapeutic agents. *Advanced Drug Delivery Reviews,* 1997, vol. 26 (2-3), 91-104
- **TEW K. D.** Glutathione-associated Enzymes in Anticancer Drug Resistance. *Cancer Research,* 1994, vol. 54 (16), 4313-4320
- **TEW K. D.** TLK-286: a novel glutathione S-transferase-activated prodrug. *Expert Opinion on Investigational Drugs,* 2005, vol. 14 (8), 1047-1054
- **TEW K. D.** Redox in redux:Emergent roles for glutathione S-transferase P (GSTP) in regulation of cell signaling and S-glutathionylation. *Biochemical Pharmacology,* 2007, vol. 73 (9), 1257-1269
- **TEW K. D. ; BOMBER A. M. et al.** Ethacrynic Acid and Piriprost as Enhancers of Cytotoxicity in Drug Resistant and Sensitive Cell Lines. *Cancer Research,* 1988, vol. 48 (13), 3622-3625
- **TIDEFELT U. ; ELMHORN-ROSENBORG A. et al.** Expression of glutathione transferase pi as a predictor for treatment results at different stages of acute nonlymphoblastic leukemia. *Cancer Res,* 1992, vol. 52 (12), 3281-3285
- **TOWNSEND D. M. ; TEW K. D.** The role of glutathione-S-transferase in anti-cancer drug resistance. *Oncogene,* 2003, vol. 22 (47), 7369-7375
- **TURELLA P. ; CERELLA C. et al.** Proapoptotic Activity of New Glutathione S-Transferase Inhibitors. *Cancer Research,* 2005, vol. 65 (9), 3751-3761
- **WANG T. ; ARIFOGLU P. et al.** Glutathione S-transferase P1-1 (GSTP1-1) inhibits c-Jun N-terminal kinase (JNK1) signaling through interaction with the C terminus. *J Biol Chem,* 2001, vol. 276 (24), 20999-21003
- **ZHENG J. ; LIU, G. ; TOZKOPARAN, B. ; WEN, D.** Mechanistic Studies of Inactivation of Glutathione S-transferase Pi Isozyme by a Haloenol Lactone Derivative. *Medicinal Chemistry,* 2005, vol. 1 (2), 191-198
- **TU GG ; YAN YG ; CHEN XY ; LV QL ; WANG JQ ; LI SH.** Synthesis and antiproliferative assay of 1,3,4-oxadiazole and 1,2,4-triazole derivatives in cancer cells. *Drug Discoveries & Therapeutics,* 2013, vol. 7 (2), 58-65
- **GADEGONI H ; MANDA S.** Synthesis and screening of some novel substituted indoles contained 1,3,4-oxadiazole and 1,2,4-triazole moiety. *Chinese Chemical Letters,* 2013, vol. 24, 127-130
- **PLECH T ; WUJEC M ; KOSIKOWSKA U ; MALM A ; RAJTAR B ; POLZ-DACEWICZ M.** Synthesis and in vitro activity of 1,2,4-triazole-ciprofloxacin hybrids against drug-susceptible and drug-resistant bacteria. *European Journal of Medicinal Chemistry,* 2013, vol. 60, 128-134
- **WITTINE K ; STIPKOVIĆ BABIĆ M ; MAKUĆ D ; PLAVEĆ J ; KRALJEVIĆ PAVELIĆ S ; SEDIĆ M ; PAVELIĆ K ; LEYSSEN P ; NEYTS J ; BALZARINI J.** Novel 1,2,4-triazole and imidazole derivatives of L-ascorbic and imino-ascorbic acid: synthesis, anti-HCV and antitumor activity evaluations. *Bioorganic & Medicinal Chemistry,* 2012, vol. 20, 3675-3685
- **PLECH T ; LUSZCZKI JJ ; WUJEC M ; FLIEGER J ; PIZOŃ M.** Synthesis, characterization and preliminary anticonvulsant evaluation of some 4-alkyl-1,2,4-triazoles. *European Journal of Medicinal Chemistry,* 2013, vol. 60, 208-215
- **PLECH T ; WUJEC M ; SIWEK A ; KOSIKOWSKA U ; MALM A.** Synthesis and antimicrobial activity of thiosemicarbazides, s-triazoles and their Mannich bases bearing 3-chlorophenyl moiety. *European Journal of Medicinal Chemistry,* 2011, vol. 46, 241-248
- **SHIVARAMA HOLLA B ; VEERENDRA B ; SHIVANANDA MK ; POOJARY B.** Synthesis characterization and anticanceractivitystudies on some Mannichbasesderived from 1,2,4-triazoles. *European Journal of Medicinal Chemistry,* 2003, vol. 38, 759-767
- **MOULIN A ; BRUNEL L ; BOEGLIN D ; DEMANGE L ; RYAN J ; M'KADMI C ; DENOYELLE S ; MARTINEZ J ; FEHRENTZ JA.** The 1,2,4-triazole as a scaffold for the design of ghrelin receptor ligands: development of JMV 2959, a potent antagonist. *Amino Acids,* 2013, vol. 44, 301-314